# EUROPEAN PATENT APPLICATION

(11) **EP 4 775 596 A1**
(43) Date of publication of application: **15.07.2026**
(21) Application number: 24862092.4
(22) Date of filing: 06.09.2024
(51) Int. Cl.: C07K 16/30, A61K 39/395, C12N 15/13, A61P 35/00

(54) **CYSTEINE ENGINEERED ANTIBODY AND IMMUNOCONJUGATE**

(30) Priority: 08.09.2023 CN 202311156907; 05.09.2024 CN 202411239766
(71) Applicant: Innovent Biologics (Suzhou) Co., Ltd., Suzhou, Jiangsu 215123 (CN)
(72) Inventor: ZHAO, Menghui, Suzhou, Jiangsu 215123 (CN); ZHU, Chenchen, Suzhou, Jiangsu 215123 (CN); GAO, Changshou, Suzhou, Jiangsu 215123 (CN)
(74) Representative: Sagittarius IP
(86) International application number: PCT/CN2024/117511
(87) International publication number: WO 2025/051254

(57) **Abstract**

The present invention relates to antibodies engineered with reactive cysteine residues, and more particularly, the present invention relates to antibodies having therapeutic or diagnostic applications. The present invention also relates to immunoconjugates comprising the engineered antibodies. The present invention further relates to a medicament or a pharmaceutical composition comprising the antibodies or antibody-drug conjugates, and methods and uses of applying the antibodies or immunoconjugates in the treatment of a disease such as a tumor.

## Description

The present invention relates to an antibody engineered with reactive cysteine residues, and more particularly, the present invention relates to an antibody having therapeutic or diagnostic applications. The present invention also relates to an immunoconjugate comprising the engineered antibodies. The present invention further relates to a medicament or a pharmaceutical composition comprising the antibodies or immunoconjugates, and a method and use of applying the antibodies or immunoconjugates in the treatment of a disease such as a tumor.

### Background

Despite the clinical success of therapeutic antibodies, naked antibodies that target cell surface tumor antigens rarely provide sufficient efficacy alone. To increase the low activity of antibodies, new strategies focus on binding the antibodies to toxic molecules. Plant and bacterial toxins and small chemotherapeutic molecules may be good candidates because they are very effective and active in very small amounts. Therefore, the conjugation of antibodies to other components to form new immunoconjugates, such as Antibody-Drug Conjugates (ADCs), has become a new strategy for the development of tumor therapy.

At present, most of the methods for conjugation of ADC are still random conjugation, including cysteine conjugation and lysine conjugation, etc., which will cause unhomogeneous distribution of ADC products. For example, the cysteine-conjugated ADC with an average DAR value of 4 comprises multiple components from DAR0 to DAR8. Even in components with the same DAR value, there are still differences in the conjugation at different sites. While these components tend to have different properties. For example, the DAR0 component will competitively bind to the target, and the DAR8 component is prone to aggregation due to the large number of hydrophobic drugs conjugated, making it easier to be cleared in the body. Different pharmacodynamic and pharmacokinetic characteristics corresponding to each of these components make PK/PD analysis of heterogeneous ADC mixtures more difficult, and a higher manufacturing process is required to produce relatively stable batches of ADC products.

In order to obtain a homogeneous ADC product, Genentech tried to mutate a site of the antibody to cysteine in 2006 (WO2006034488A2) so that the drug could be conjugated homogeneously to this mutated site. Cysteine introduced into the antibody screened by the patent has higher thiol reactivity, and a mutation to cysteine does not affect the binding capacity of the antibody to its antigen, and in addition, theADC has stronger efficacy in vivo than that of the randomly conjugated ADC. However, there are still some limitations in this technology.

With the increasing research in recent years, it was found that some diseases or targets are far from reaching the expected efficacy by only conjugating 2 drugs, so the technology for obtaining a homogeneous product with higher DAR is also important.

The strategies for screening the engineered sites disclosed in the prior art are relatively limited. In order to screen sites with higher thiol reactivity and to obtain ADCs with higher DAR, the existing selection on the sites has a bias towards the sites exposure to the antibody surface, thus having higher solvent accessibility. However, a series of other negative effects brought by sites with high solvent accessibility, such as ADC stability after drug conjugation, hydrophilicity and hydrophobicity, PK behavior, in vivo efficacy, etc., are not considered.

Therefore, there is still a need in the art for a new cysteine-engineering technology for antibodies, based on which the obtained antibodies can be used to construct ADCs with greater stability and greater efficacy.

### Summary of the Invention

The present invention relates to a modified antibody or antigen-binding fragment thereof having mutations to cysteine at cryptic sites of the constant region, thereby conferring greater stability and/or hydrophilicity to an immunoconjugate comprising the same.

In one embodiment, the cysteine-engineered antibody of the present invention, after being mutatated for one or two amino acids to cysteines on its light chain or heavy chain, will result in two or four cysteine-engineered antibody molecules due to the dimerization property of IgG antibodies. The thiol group on the cysteine can undergo a nucleophilic reaction with a toxin small molecule having a maleimide linker, thereby preparing a site-specifically conjugated ADC molecule by conjugation of the toxin small molecule to the four cysteines. The selection of mutation sites is the key point in the present invention, and the quality of the sites directly determines the properties of the ADC. After the small molecule drug is linked to a site with high solvent accessibility, the possibility of thiol exchange between the small molecule and the protein in the plasma is increased due to the relatively exposed environment of the small molecule drug, thus weakening the efficacy and toxic and side effects. Furthermore, the more exposed small molecules also significantly increase the hydrophobicity of the antibody, thus affecting PK behavior in vivo of the antibody. Thus, the selection of more cryptic sites for engineering may result in more stable and more effective ADC molecules.

The present invention also relates to an immunoconjugate comprising the modified antibody or antigen-binding fragment thereof. The immunoconjugate of the present invention may have a DAR of 4, so that the need for more targets or diseases can be met. In some embodiments, the immunoconjugate is an Antibody-Drug Conjugate (ADC).

The ADCs of the present invention are site-directed conjugated ADCs with better physicochemical properties, which have one or more of the following advantages compared to randomly conjugated DAR4 ADCs:
(1) more hydrophilicity,
(2) more stability in plasma and the small molecular toxin is not easy to shed;
(3) greater efficacy in animals, for example, the ADC with a DAR of 4 of the present invention can be comparable effective to that of known ADCs with a DAR of 8.

### DESCRIPTION OF THE FIGURES:

FIG. 1 shows a representative diagram for the analysis and calculation of the site-specific conjugating DAR values.
FIG. 2 shows a representative diagram for the analysis and calculation of random conjugating DAR values.
FIG. 3 shows a representative diagram for the purity analysis of LLC 166-HC 155-Dxd.
FIG. 4 shows a hydrophobic interaction spectrum for each DAR2 ADC.
FIG. 5 shows a hydrophobic interaction spectrum for each DAR4 ADC.
FIGs. 6A and 6B show the results for cell viability in vitro cell proliferation experiments.
FIGs. 7A and 7B show the fluorescence values of each ADC binding to SK-BR-3 in vitro detected by flow cytometry.
FIG. 8A shows the change in DAR for each ADC with DAR2 in mouse plasma, and FIGs. 8B and 8C show the change in DAR for each ADC with DAR4 in mouse plasma.
FIGs. 9A-E show the inhibitory effect of each ADC on mouse tumors at certain doses (where FIGs. 9A-C and E show intraperitoneal administration and FIG. 9D shows intravenous administration).

### DETAILED DESCRIPTION OF THE INVENTION

### I. Definitions

Before the present invention is described in detail below, it is to be understood that the present invention is not limited to the particular methodology, protocols, and reagents described herein, as these may vary. It is also to be understood that the terms used herein is for the purpose of describing particular embodiments only, and is not intended to limit the scope of the present invention which will be limited only by the appended claims. Unless otherwise specified, all technical and scientific terms used herein have the same meanings as those generally understood by a person of ordinary skill in the art to which the present invention belongs.

For purposes of interpreting this specification, the following definitions will be used, and where appropriate, terms used in the singular may also include the plural, and vice versa. It is to be understood that the terms used herein are for the purpose of describing particular embodiments only, and are not intended to be limiting.

The term "about" when used in conjunction with a numerical value is intended to encompass the numerical value within a range having a lower limit that is 5% less than the numerical value specified, and an upper limit that is 5% greater than the numerical value specified.

As used herein, the term "and/or" means any one of the alternatives or two or more of the alternatives.

As used herein, the terms "comprising" or "including" means including the recited elements, integers, or steps, but not excluding any other elements, integers, or steps. When the term "comprising" or "including" is used herein, unless otherwise indicated, it also encompasses the situation combining the stated elements, integers, or steps. For example, reference to an antibody variable region "comprising" the particular sequence is also intended to encompass the antibody variable regions consisting of that particular sequence.

The terms "complete antibody," "whole antibody," or "full-length antibody" are used interchangeably herein to refer to an antibody molecule having the structure of a native immunoglobulin molecule. In the case of a conventional four-chain IgG antibody, the full-length antibody comprises two heavy chains (H) and two light chains (L) interconnected by disulfide bonds. In the case of a heavy chain antibody having only heavy chains and lacking light chains, the full-length antibody comprises two heavy chains (H) interconnected by disulfide bonds. For conventional four-chain IgG antibodies, the full-length antibody heavy chain typically consists of a heavy chain variable region (abbreviated herein as VH) and a heavy chain constant region, wherein the heavy chain constant region comprises at least 3 domains CH1, CH2, and CH3. A full-length antibody light chain consists of a light chain variable region (abbreviated herein as VL) and a light chain constant region, wherein the light chain constant region consists of one domain CL. Each heavy chain variable region VH and each light chain variable region consists of three CDRs and four FRs, arranged from amino-terminus to carboxy-terminus in the following order: FR1, CDR1, FR2, CDR2, FR3, CDR3, FR4.

The constant regions are not directly involved in binding of antibodies to antigens, but exhibit a variety of effector functions. In some embodiments, the antibody heavy chain constant region HC of the present invention is the heavy chain constant region of IgG 1, IgG2, IgG3 or IgG4, preferably the heavy chain constant region of IgG1. In some preferred embodiments, the heavy chain constant region HC of the present invention
(i) comprises or consists of an amino acid sequence having at least 85%, 90%, 91%, 92%, 93%, 94%, 95%, 96%, 97%, 98%, or 99% identity to the amino acid sequence of SEQ ID NO: 3;
(ii) comprises or consists of an amino acid sequence of SEQ ID NO: 3; or
(iii) comprises or consists of an amino acid sequence having 1 or more (preferably not more than 20 or 10, more preferably not more than 5, 4, 3, 2, 1) amino acid alterations (preferably amino acid replacements, more preferably amino acid conservative replacements) compared to the amino acid sequence of SEQ ID NO: 3.

In some embodiments, the antibody light chain constant region LC of the present invention is a Lambda or Kappa light chain constant region. In some embodiments, the antibody light chain constant region LC of the present invention
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 5 or 6;
(ii) comprises or consists of an amino acid sequence selected from SEQ ID NO: 5 or 6; or
(iii) comprises or consists of an amino acid sequence having 1 or more (preferably not more than 20 or 10, more preferably not more than 5, 4, 5 or 3, 2, 1) amino acid alterations (preferably amino acid replacements, more preferably amino acid conservative replacements) compared to the amino acid sequence selected from SEQ ID NO: 5 or 6.

The term "CH1 region" refers to the portion of an antibody heavy chain polypeptide that extends from EU position 118 to EU position 220 (EU numbering system). In one embodiment, the CH1 domain comprises the amino acid sequence of

ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSC (SEQ ID NO: 1).

The term "Fc domain" or "Fc region" is used herein to define a C-terminal region of an immunoglobulin heavy chain that contains at least a portion of a constant region. This term includes native sequence Fc regions and variant Fc regions. A native immunoglobulin "Fc domain" comprises two or three constant domains, namely the CH2 domain, the CH3 domain, and the optional CH4 domain. For example, in natural antibodies, the immunoglobulin Fc domain comprises the second and third constant domains (CH2 and CH3 domains) derived from the two heavy chains of IgG, IgA, and IgD class antibodies; or comprises the second, third, and fourth constant domains (CH2, CH3, and CH4 domains) derived from the two heavy chains of the IgM and IgE classes antibodies. Unless otherwise indicated herein, the amino acid residues in the Fc region or heavy chain constant region are numbered according to the EU numbering system (also known as the EU index) as described in Kabat et al., Sequences of Proteins of Immunological Interes, 5th Edition, Public Health Service, National Institutes of Health, Bethesda, MD, 1991. However, the C-terminal lysine (Lys447) of the Fc region may or may not be present. Two Fc regions can be dimerized to form a dimeric Fc, for example, two different Fcs can be heterodimerized to form a heterodimeric Fc. In this context, the terms "Fc region", "Fc portion" and "dimeric Fc (e.g., heterodimeric Fc)" do not include the heavy chain variable region VH and the light chain variable region VL and the heavy chain constant region CH1 and the light chain constant region CL of an immunoglobulin, but may include a hinge region at N-terminal of the heavy chain constant region in some cases. In one embodiment, the human IgG heavy chain Fc region extends from Asp221 or from Cys226 or from Asp231 to the carboxy-terminus of the heavy chain.

In one embodiment, a human IgG1 Fc region polypeptide (comprising a hinge region) comprises the following amino acid sequence:

DKTHTCPPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQQGNVFSCSV
MHEALHNHYTQKSLSLSPGK (SEQ ID NO: 2)。

The human IgG4 Fc region polypeptide (comprising a hinge region) comprises the following amino acid sequence:

PPCPSCPAPEFLGGPSVFLFPPKPKDTLMISRTPEVTCVVVDVSQEDPEVQFNWYVDGVEVHNAKTK
PREEQFNSTYRVVSVLTVLHQDWLNGKEYKCKVSNKGLPSSIEKTISKAKGQPREPQVYTLPPSQEEMTK
NQVSLTCLVKGFYPSDIAVEWESNGQPENNYKTTPPVLDSDGSFFLYSRLTVDKSRWQEGNVFSCSVMHE
ALHNHYTQKSLSLSLGK (SEQ ID NO: 4).

The term "antibody fragment" encompasses a portion of an intact antibody. In a preferred embodiment, the antibody fragment is an antigen-binding fragment.

The term "antigen-binding fragment" of an antibody is a molecule distinct from a full-length antibody, which comprises a portion of the full-length antibody, but which binds to an antigen of the full-length antibody or competes for binding to an antigen with the full-length antibody (i.e., with the full-length antibody from which the antigen-binding fragment is derived). The antigen-binding fragments may be prepared by recombinant DNA techniques, or by enzymatic or chemical cleavage of intact antibodies. antigen-binding fragments include, but are not limited to, Fab, Fab', F(ab')2, Fv, single chain Fv, diabodies, single domain antibodies (sdAb), nanobodies. For example, Fab fragments may be obtained by papain digestion of full-length antibodies. In addition, digestion of the complete antibody by pepsin below the disulfide bond in the hinge region produces F(ab')2, which is a dimer of Fab' and is a bivalent antibody fragment. The F(ab')2 may be reduced under neutral conditions by disrupting the disulfide bond in the hinge region, thereby converting the F(ab')2 dimer into a Fab' monomer. The Fab' monomer is essentially a Fab fragment with a hinge region. The Fv fragment consists of the VL and VH domains of a single arm of an antibody. The two domains of the Fv fragment, VL and VH, may be encoded by separate genes, but it is also possible to link the two domains by recombinant methods using a synthetic linker peptide so that they are produced as a single protein chain in which the VL region and the VH region are paired to form a single chain Fv (scFv). "Fab fragment" or "Fab" are used interchangeably herein to refer to an immunoglobulin fragment consisting of two polypeptide chains, comprising a heavy chain variable domain VH, a heavy chain constant domain CH1, a light chain variable domain VL and a light chain constant domain CL of an immunoglobulin, wherein one polypeptide chain comprises, from N-terminus to C-terminus, VH and one constant region selected from CH1 and CL, and the other polypeptide chain comprises, from N-terminus to C-terminus, VL and another constant region selected from CL and CH1, wherein the VH and VL domains are paired to form an antigen-binding site. In this context, the Fab polypeptide chain comprising the heavy chain constant region CH1 is also referred to as the "Fab heavy chain"; accordingly, the Fab polypeptide chain comprising the light chain constant region CL is also referred to as "Fab light chain".

The term "antigen" refers to a molecule that elicits an immune response. Such an immune response may involve antibody production or activation of specific immune cells, or both. Those skilled will appreciate that any macromolecule, including substantially all proteins or peptides, may be used as an antigen. Furthermore, the antigen may be derived from recombinant or genomic DNA. As used herein, the term "epitope" refers to a portion of an antigen that specifically interacts with an antibody molecule.

A "complementary determining region" or "CDR region" or "CDR" is a region of an antibody variable domain that is highly variable in sequence and forms a structurally defined loop ("hypervariable loop") and/or includes an antigencontact residue ("antigencontact point"). The CDRs are primarily responsible for the binding to antigenic epitopes. The CDRs of the heavy chain and light chain are generally referred to as CDR1, CDR2, and CDR3, numbered sequentially from the N-terminus. CDRs located within the variable domain of the heavy chain of an antibody are referred to as HCDR1, HCDR2, and HCDR3, while CDRs located within the variable domain of the light chain of an antibody are referred to as LCDR1, LCDR2, and LCDR3. In a given light chain variable region or heavy chain variable region amino acid sequence, the exact amino acid sequence boundaries of each CDR may be determined using any one or a combination of a number of well-known antibody CDR assignment systems, including, for example: Chothia based on the three-dimensional structure of antibodies and the topology of CDR loops (Chothia et al., (1989) Nature 342: 877-883; Al-Lazikani et al., "Standard conformations for the canonical structures of immunoglobulins", Journal of Molecular Biology, 273, 927-948 (1997)); Kabat based on antibody sequence variability (Kabat et al., Sequences of Proteins of Immunological Interest, 4th Edition, U.S. Department of Health and Human Services, National Institutes of Health (1987), AbM (University of Bath), Contact (University College London), International ImmunoGeneTics database (IMGT)(available on www. imgt. cines. fr/); and a North CDR definition based on affinity propagation clustering using a large number of crystal structures (North et al., "A New Clustering of Antibody CDR Loop Conformations", Journal of Molecular Biology, 406, 228-256(2011)).

Unless otherwise indicated, in the present invention, the term "CDR" or "CDR sequence" encompasses CDR sequences determined in any of the ways described above.

As used herein, when referring to amino acid positions in antibody domains other than the variable region (e.g., the constant region, e.g., the Fc region), the position is numbered according to the EU numbering system (also known as the EU index) as described in Kabat et al., Sequences of Proteins of Immunological Interes, 5th Edition, Public Health Service, National Institutes of Health, Bethesda, MD, 1991. When position numbering and/or amino acid residues are assigned to a particular antibody isotype, it is intended to apply to the corresponding position and/or amino acid residue of any other antibody isotype, as is known to those skilled in the art.

General information on the human immunoglobulin light chain and heavy chain is described in Kabat, E.A. et al., Sequences of Proteins of Immunological Interest, 5th Edition, Public Health Service, National Institutes of Health, Bethesda, MD (1991).

The term "amino acid substitution" or "amino acid mutation" refers to the replacement of at least one amino acid residue in a predetermined parent amino acid sequence with different "substitution" amino acid residues. The substitution residue or residues may be "naturally occurring amino acid residues" (i.e., encoded by the genetic code) and are selected from: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val).

The amino acid position to be mutated to cysteine is generally indicated by "chain type, mutation position". In this context, LLC represents the Lambda light chain, LC represents the Kappa light chain, and HC represents the heavy chain, unless otherwise specified. Thus, "LLC160" means that the amino acid at EU position 160 of the Lambda light chain is substituted with cysteine (C). "LC165" means that amino acid 165 at the EU position of the Kappa light chain is substituted with cysteine (C). When referring to a combination of mutations, the combined mutations are joined by a plus (-). "LLC160-LLC166" means a mutation to cysteine at position 160 of LLC and a mutation to cysteine at position LLC 166 are both included.

When the present invention refers to heavy chain amino acid positions, it refers to the amino acid positions numbered based on the IgG1 heavy chain, i.e., it encompasses the amino acid positions numbered based on the IgG1 heavy chain and the amino acid positions on other heavy chains corresponding to said amino acid positions, unless otherwise specified. For example, when referring to HC290, it encompasses amino acid position 290 of the IgG1 heavy chain under EU numbering.

"Sequence identity" is defined as the percentage of identical residues in an amino acid sequence variant after aligning the sequences and introducing gaps, if necessary, to achieve the maximum percent sequence identity.

A "free cysteine" refers to a cysteine residue that has been engineered into a parent antibody, has a thiol functional group (-SH) (sulfhydryl group), and does not pair or otherwise become part of an intramolecular or intermolecular disulfide bridge.

A "parent protein (e.g., a parent antibody or a parent constant region or a parent Fc region)" refers to a protein comprising an amino acid sequence in which one or more amino acid residues are to be replaced with one or more cysteine residues. A parent protein may comprise a native or wild-type sequence. A parent protein may have existing amino acid sequence modifications (such as additions, deletions, and/or substitutions) relative to other native, wild-type, or modified proteins. A parent antibody may be directed against a target antigen of interest, such as a biologically important polypeptide. In some embodiments, the parent antibody is an antibody against HER2, such as trastuzumab.

The terms "host cell," "host cell line," and "host cell culture" are used interchangeably and refer to a cell into which an exogenous nucleic acid has been introduced, including progeny of such a cell. Host cells include "transformants" and "transformed cells," which include the primary transformed cells and progeny derived therefrom, regardless of the number of passages. The progeny may not be exactly the same as the parent cell in nucleic acid content, but may contain mutations. Included herein are mutant progeny that have the same function or biological activity as screened or selected for the originally transformed cell.

The term "vector" as used herein refers to a nucleic acid molecule capable of propagating another nucleic acid to which it is linked. The term includes vectors which are self-replicating nucleic acid structures as well as vectors which are incorporated into the genome of a host cell into which they have been introduced. Some vectors are capable of directing the expression of a nucleic acid to which they are operably linked. Such vectors are referred to herein as "expression vectors".

As used herein, an "Antibody-Drug Conjugate (ADC)" refers to a structure obtained by linking an antibody and a drug.

The term "site-specific conjugation" as used herein refers to the conjugation of a drug/active agent specifically to a specific site of an antibody via a linker.

The term "pharmaceutically acceptable salt" refers to a salt that retains the biological effects and properties of the ADC conjugates of the present invention, and which is not biologically or otherwise undesirable. The ADC conjugates of the present invention may exist in their pharmaceutically acceptable salt forms, including acid addition salts and base addition salts. In the present invention, pharmaceutically acceptable non-toxic acid addition salts refer to the salts formed by the ADC conjugates of the present invention with organic or inorganic acids including, but not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, and the like. Pharmaceutically acceptable non-toxic base addition salts refer to the salts formed by the ADC conjugates of the present invention with organic or inorganic bases including, but not limited to alkali metal salts, such as lithium salts, sodium salts or potassium salts; alkaline earth metal salts, such as calcium or magnesium salts; organic base salts, for example ammonium salts, formed with organic bases which contains N containing groups.

The term "solvate" refers to an association of one or more solvent molecules with ADC conjugates of the present invention. Solvents that form solvates include, but are not limited to, water, methanol, ethanol, isopropanol, ethyl acetate, tetrahydrofuran, N, N-dimethylformamide, dimethylsulfoxide, and the like.

"Pharmaceutically acceptable" and "pharmaceutical" are used interchangeably herein, without contradictory context.

The term "drug:antibody ratio" or "DAR" refers to the ratio of the payload moiety (D) conjugated to the antibody moiety (A) described herein to the antibody moiety. DAR may also be calculated as the average DAR of the molecular population in the product, i.e., the overall ratio of the small molecule drug moiety (D) conjugated to the Ab moiety described herein to the Ab moiety in the product as measured by detection methods (e.g., by conventional methods such as mass spectrometry, ELISA assays, electrophoresis, and/or HPLC), such DAR being referred to herein as average DAR. For example, some immunoconjugate components with a ratio of payload to antibody of 2 in the sample may have no or only one payload attached; other immunoconjugate components in the sample will have two, three, four or even more moieties on the individual antibodies. But the average value in the sample will be 2.

As an exemplary embodiment, an immunoconjugate having a "DAR of about 2" refers to an immunoconjugate in which the payload-to-antibody ratio may vary ranging from about 1.4-2.4 payload/antibody, 1.5-2.1 payload/antibody, or 1.5-1.9 payload/antibody. An immunoconjugate having a "DAR of about 4" refers to an immunoconjugate in which the payload-to-antibody ratio may vary in a range of about 3-4.5, e.g., 3-4 payload/antibody.

The term "therapeutic agent" as used herein encompasses any substance effective in preventing or treating a tumor, such as cancer, including chemotherapeutic agents, cytokines, angiogenesis inhibitors, cytotoxic agents, other antibodies, small molecule drugs, or immunomodulators (e.g., immunosuppressants).

The term "cytotoxic agent" as used herein refers to a substance that inhibits or prevents cell function and/or causes cell death or destruction.

A "chemotherapeutic agent" includes a chemical compound useful in the treatment of cancer or diseases of immune system.

The term "small molecule drug" refers to an organic compound with low molecular weight capable of modulating biological processes. A "small molecule" is defined as a molecule having a molecular weight of less than 10 kD, typically less than 2 kD, and preferably less than 1 kD. Small molecules include, but are not limited to, inorganic molecules, organic molecules, organic molecules containing inorganic components, molecules containing radioactive atoms, synthetic molecules, peptidomimetics, and antibody mimetics. As a therapeutic agent, small molecules may be more permeable to cells, less susceptible to degradation, and less prone to eliciting an immune response than large molecules.

The term "immunomodulator" as used herein refers to a native or synthetic active agent or drug that inhibits or modulates an immune response. The immune response may be a humoral response or a cellular response. The immunomodulator comprises an immunosuppressant. In some embodiments, an immune modulator of the present invention comprises an immune checkpoint inhibitor or an immune checkpoint agonist.

The term "effective amount" refers to an amount or dose of an antibody or fragment or composition or combination of the present invention which, upon administration to a patient in a single or multiple doses, produces the desired effect in the patient in need of treatment or prevention.

A "therapeutically effective amount" refers to an amount effective, at dosages and for periods of time required, to achieve the desired therapeutic result. A therapeutically effective amount is also one in which any toxic or adverse effect of the antibody or antibody fragment or composition or combination is less than a therapeutically beneficial effect. A "therapeutically effective amount" preferably inhibits a measurable parameter (e.g., tumor volume) by at least about 30%, even more preferably at least about 40%, 45%, 50%, 55%, 60%, 65%, 70%, 75%, 80%, 85%, 90% or even 100% relative to untreated subjects.

A "prophylactically effective amount" refers to an amount effective, at dosages and for periods of time required, to achieve the desired prophylactic result. Generally, a prophylactically effective amount will be less than a therapeutically effective amount because a prophylactic dose is used in a subject prior to or at an earlier stage of the disease.

As used herein, the term "label" refers to a compound or composition that is directly or indirectly conjugated or fused to an agent, such as a polynucleotide probe or antibody, and facilitates the detection of the agent to which it is conjugated or fused. The label may be detectable by itself (e.g., a radioisotope label or a fluorescent label) or, in the case of an enzymatic label, may catalyze chemical alteration of the detectable substrate compound or composition. The term is intended to encompass direct labeling of the probe or antibody by coupling (i.e., physically linking) a detectable substance to the probe or antibody, as well as indirect labeling of the probe or antibody by reaction with another reagent that is directly labeled.

An "individual" or "subject" includes mammals. Mammals include, but are not limited to, domestic animals (e.g., cows, sheep, cats, dogs, and horses), primates (e.g., humans and non-human primates such as monkeys), rabbits, and rodents (e.g., mice and rats). In some embodiments, the individual or a subject is a human.

An "isolated" antibody or other molecule (e.g., an ADC molecule) is one that has been isolated from a component of its natural environment or the environment in which it is expressed. In some embodiments, the antibody or ADC molecule is purified to greater than 95% or 99% purity as determined, for example, by electrophoresis (e.g., SDS-PAGE, Isoelectric focusing (IEF), capillary electrophoresis) or chromatography (e.g., ion exchange or reverse phase HPLC).

The term "anti-tumor effect" refers to a biological effect that can be exhibited by a variety of means, including, but not limited to, for example, a reduction in tumor volume, a reduction in tumor cell number, a reduction in tumor cell proliferation, or a reduction in tumor cell survival.

The terms "tumor" and "cancer" are used interchangeably herein to encompass both solid tumors and hematological tumors.

The terms "cancer" and "cancerous" refer to or describe a physiological disease in mammals that is typically characterized by unregulated cell growth. In certain embodiments, cancers suitable for treatment by the antibodies of the present invention include gastric, pancreatic, or gastroesophageal junction cancers, including metastatic forms of those cancers.

The term "tumor" refers to all neoplastic cell growth and proliferation, whether malignant or benign, and all pre-cancerous and cancerous cells and tissues. The terms "cancer," "cancerous," and "tumor" mentioned herein are not mutually exclusive.

The term "pharmaceutical excipient" refers to a diluent, adjuvant (e.g., Freund's adjuvant (complete and incomplete)), vehicle, carrier or stabilizer, etc., with which the active substance is administered.

The term "pharmaceutical composition" refers to a composition that is present in a form that allows the biological activity of the active ingredients contained therein to be effective, and which does not contain additional ingredients having unacceptable toxicity to the subject to which the composition is administered.

The term "pharmaceutical combination" or "pharmaceutical combination product" or "combination product" refers to a non-fixed combination product or a fixed combination product, including but not limited to a kit, a pharmaceutical composition. The term "non-fixed combination" means that the active ingredients (e.g., (i) an antibody or immunoconjugate molecule of the present invention, and (ii) other therapeutic agents) are administered to a patient as separate entities either simultaneously, without specific time limitations, or sequentially at the same or different time intervals, wherein such administration provides prophylactically or therapeutically effective levels of the two or more active agents in the patient. In some embodiments, the antibody or immunoconjugate molecule of the present invention and the other therapeutic agents used in the pharmaceutical combination are administered at a level no greater than that when they are used alone. The term "fixed combination" means that two or more active agents are administered to a patient simultaneously in the form of a single entity. The dosage and/or time intervals of the two or more active agents are preferably selected such that the combined use of the components results in a greater effect in the treatment of the disease or disorder than that would be achieved by use of either component alone. The ingredients may each be in separate form of formulation, which may be the same or different.

The term "combination therapy" refers to the administration of two or more therapeutic agents or treatment modalities (e.g., radiation therapy or surgery) to treat the diseases described herein. Such administration includes co-administering the therapeutic agents in a substantially simultaneous manner, for example, in a single capsule having a fixed ratio of active ingredients. Alternatively, such administration includes co-administration of the active ingredients in multiple or separate containers (e.g., tablets, capsules, powders, and liquids). The powder and/or liquid may be reconstituted or diluted to the desired dosage prior to administration. In addition, such administration also includes the use of each type of therapeutic agent at approximately the same time or at different times in a sequential manner. In either case, the treatment regimen will provide a beneficial effect of the drug combination in treating the disorders or conditions described herein.

As used herein, "treating" refers to slowing, interrupting, arresting, relieving, stopping, reducing, or reversing the progression or severity of an existing symptom, disorder, condition, or disease.

As used herein, "preventing" includes the inhibition of the occurrence or development of a disease or disorder or the symptoms of a particular disease or disorder. In some embodiments, a subject with a family history of caner is a candidate for a prophylactic regimen. In general, in the context of caner, the term "prevention" refers to the administration of a drug prior to the onset of a sign or symptom of caner, particularly in a subject at risk for caner.

The term "vector" as used herein refers to a nucleic acid molecule capable of propagating another nucleic acid to which it is linked. The term includes vectors which are self-replicating nucleic acid structures as well as vectors which are incorporated into the genome of a host cell into which they have been introduced. Some vectors are capable of directing the expression of a nucleic acid to which they are operably linked. Such vectors are referred to herein as "expression vectors".

A "subject/patient/individual sample" refers to a collection of cells or fluids obtained from a patient or subject. The source of the tissue or cell sample may be a solid tissue, such as fresh, frozen and/or preserved organs or tissue samples or biopsy samples or puncture samples; blood or any blood component; body fluids such as cerebrospinal fluid, amniotic fluid, peritoneal fluid (ascites), or interstitial fluid; cells from a subject at any time of pregnancy or development. Tissue samples may contain compounds that are naturally not intermixed with tissue in nature, such as preservatives, anticoagulants, buffers, fixatives, nutrients, antibiotics, etc.

### II. Cysteine-engineered antibody

In some embodiments, the present invention provides a modified antibody or antigen-binding fragment thereof, wherein the modified antibody or antigen-binding fragment thereof comprises substitution(s) of one or more amino acids with cysteine at position(s) selected from position 152, 155, or 290 of the heavy chain constant region as compared to a parent antibody or antigen-binding fragment thereof.

In some embodiments, the present invention provides a modified antibody or antigen-binding fragment thereof, wherein the modified antibody or antigen-binding fragment thereof comprises substitution(s) of one or more amino acids with cysteine at position(s) selected from position 138, 160, 165, 166, or 167 of the light chain constant region as compared to a parent antibody or antigen-binding fragment thereof. In some embodiments, when the light chain constant region is a Kappa light chain constant region, it may further comprise a 178Y mutation at position 178, i.e., the amino acid at position 178 is mutated to tyrosine Y.

In some embodiments, the present invention provides a modified antibody or antigen-binding fragment thereof, wherein the modified antibody or antigen-binding fragment thereof comprises substitution(s) of one or more (e.g., 2, 3, or 4) amino acids with cysteine at position(s) selected from position 152, 155, or 290 of the heavy chain constant region or at positions selected from position 138, 160, 165, 166, or 167 of the light chain constant region as compared to a parent antibody or antigen-binding fragment thereof.

In some embodiments, the heavy chain constant region is a heavy chain constant region of IgG1, IgG2, IgG3 or IgG4, for example, a heavy chain constant region of IgG1. In some embodiments, the light chain constant region is a Kappa light chain constant region or a Lambda light chain constant region.

In some embodiments, the present invention provides a modified antibody or antigen-binding fragment thereof, wherein the modified antibody or antigen-binding fragment thereof comprises mutations to cysteine at positions 160 and 166 of the light chain constant region as compared to a parent antibody or antigen-binding fragment thereof. In some embodiments, the light chain constant region is a Lambda light chain constant region or a Kappa light chain constant region, preferably a Lambda light chain constant region.

In some embodiments, the present invention provides a modified antibody or antigen-binding fragment thereof, wherein the modified antibody or antigen-binding fragment thereof comprises mutations to cysteine at positions 160 and 166 and a mutation to tyrosine Y at position 178 of the light chain constant region as compared to a parent antibody or antigen-binding fragment thereof. In some embodiment, the light chain constant region is a Kappa light chain constant region.

In some embodiments, the present invention provides a modified antibody or antigen-binding fragment thereof, wherein the modified antibody or antigen-binding fragment thereof comprises a mutation to cysteine at position 166 of the light chain constant region and a mutation to cysteine at position 152 of the heavy chain constant region as compared to a parent antibody or antigen-binding fragment thereof. In some embodiments, the light chain constant region is a Lambda light chain constant region or a Kappa light chain constant region (preferably a Lambda light chain constant region), and/or the heavy chain constant region is an IgG1 heavy chain constant region.

In some embodiments, the present invention provides a modified antibody or antigen-binding fragment thereof, wherein the modified antibody or antigen-binding fragment thereof comprises a mutation to cysteine at position 160 of the light chain constant region and comprises a mutation to cysteine at position 155 of the heavy chain constant region as compared to a parent antibody or antigen-binding fragment thereof. In some embodiments, the light chain constant region is a Lambda light chain constant region or a Kappa light chain constant region (preferably a Lambda light chain constant region), and/or the heavy chain constant region is an IgG1 heavy chain constant region.

In some embodiments, the present invention provides a modified antibody or antigen-binding fragment thereof, wherein the modified antibody or antigen-binding fragment thereof comprises a mutation to cysteine at position 166 of the light chain constant region and comprises a mutation to cysteine at position 155 of the heavy chain constant region as compared to a parent antibody or antigen-binding fragment thereof. In some embodiments, the light chain constant region is a Lambda light chain constant region or a Kappa light chain constant region (preferably a Lambda light chain constant region), and/or the heavy chain constant region is an IgG1 heavy chain constant region.

In some embodiments, the present invention provides a modified antibody or antigen-binding fragment thereof, wherein the modified antibody or antigen-binding fragment thereof comprises a mutation to cysteine at position 165 of the light chain constant region and comprises a mutation to cysteine at position 152 of the heavy chain constant region as compared to a parent antibody or antigen-binding fragment thereof. In some embodiments, the light chain constant region is a Kappa light chain constant region or a Lambda light chain constant region (preferably a Kappa light chain constant region), and/or the heavy chain constant region is an IgG1 heavy chain constant region.

In some embodiments, the present invention provides a modified antibody or antigen-binding fragment thereof, wherein the modified antibody or antigen-binding fragment thereof comprises a mutation to cysteine at position 165 of the light chain constant region and comprises a mutation to cysteine at position 155 of the heavy chain constant region as compared to a parent antibody or antigen-binding fragment thereof. In some embodiments, the light chain constant region is a Kappa light chain constant region or a Lambda light chain constant region (preferably a Kappa light chain constant region), and/or the heavy chain constant region is an IgG1 heavy chain constant region.

In some embodiments, the modified antibody or antigen-binding fragment thereof of the present invention comprises a modified light chain constant region. In some embodiments, the modified antibody or antigen-binding fragment thereof of the present invention comprises a light chain comprising a modified light chain constant region.

In some embodiments, the modified antibody or antigen-binding fragment thereof of the present invention comprises a modified heavy chain constant region. In some embodiments, the modified antibody or antigen-binding fragment thereof of the present invention comprises a heavy chain constant region comprising a modified CH1. In some embodiments, the modified antibody or antigen-binding fragment thereof of the present invention comprises a heavy chain constant region comprising a modified Fc region. In some embodiments, the modified antibody or antigen-binding fragment thereof of the present invention comprises a heavy chain constant region comprising a modified CH1 and/or a modified Fc region. In some embodiments, the modified antibody or antigen-binding fragment thereof of the present invention comprises a modified CH1. In some embodiments, the modified antibody or antigen-binding fragment thereof of the present invention comprises a modified Fc region. In some embodiments, the modified antibody or antigen-binding fragment thereof of the present invention comprises a modified CH1 and/or a modified Fc region.

In some embodiments, the modified antibody or antigen-binding fragment thereof of the present invention comprises a modified light chain constant region and a modified heavy chain constant region. In some embodiments, the modified antibody or antigen-binding fragment thereof of the present invention comprises a modified light chain constant region and a modified CH1. In some embodiments, the modified antibody or antigen-binding fragment thereof of the present invention comprises a modified light chain constant region and a modified Fc region. In some embodiments, the modified antibody or antigen-binding fragment thereof of the present invention comprises a modified light chain constant region and a modified Fc region and/or a modified CH1.

In some embodiments, the parent human Lambda light chain constant region of the present invention
comprises or consists of the amino acid sequence set forth in SEQ ID NO: 6, or comprises an amino acid sequence having at least 90%, 91%, 92%, 93%, 94%, 95%, 96%, 97%, 98%, or 99% sequence identity to the amino acid sequence set forth in SEQ ID NO: 6 and comprising no mutation to cysteine.

In some embodiments, the parent human Kappa light chain constant region of the present invention
comprises or consists of the amino acid sequence set forth in SEQ ID NO: 5, or comprises an amino acid sequence having at least 90%, 91%, 92%, 93%, 94%, 95%, 96%, 97%, 98%, or 99% sequence identity to the amino acid sequence set forth in SEQ ID NO:5 and comprising no mutation to cysteine.

In some embodiments, the parent human IgG1 heavy chain constant region of the present invention
comprises or consists of the amino acid sequence set forth in SEQ ID NO: 3, or comprises an amino acid sequence having at least 90%, 91%, 92%, 93%, 94%, 95%, 96%, 97%, 98%, or 99% sequence identity to the amino acid sequence set forth in SEQ ID NO: 3 and comprising no mutation to cysteine.

In some embodiments, the parent human IgG4 Fc region of the present invention
comprises or consists of the amino acid sequence set forth in SEQ ID NO: 4, or comprises an amino acid sequence having at least 90%, 91%, 92%, 93%, 94%, 95%, 96%, 97%, 98%, or 99% sequence identity to the amino acid sequence set forth in SEQ ID NO: 4 and comprising no mutation to cysteine.

In some embodiments, the parent human IgG 1 Fc region of the present invention
comprises or consists of the amino acid sequence set forth in SEQ ID NO: 2, or comprises an amino acid sequence having at least 90%, 91%, 92%, 93%, 94%, 95%, 96%, 97%, 98%, or 99% sequence identity to the amino acid sequence set forth in SEQ ID NO: 2 and comprising no mutation to cysteine.

In some embodiments, the parent human IgG1 CH1 of the present invention
comprises or consists of the amino acid sequence set forth in SEQ ID NO: 1, or comprises an amino acid sequence having at least 90%, 91%, 92%, 93%, 94%, 95%, 96%, 97%, 98%, or 99% sequence identity to the amino acid sequence set forth in SEQ ID NO: 1 and comprising no mutation to cysteine.

In some embodiments, the modified light chain constant region of the present invention is a Lambda light chain constant region and comprises the amino acid sequence set forth in SEQ ID NO: 7, 8 or 28. In some embodiments, the modified light chain constant region of the present invention is a Kappa light chain constant region and comprises the amino acid sequence set forth in SEQ ID NO: 9, 12, 14 or 62.

In some embodiments, the modified light chain constant region of the present invention comprises or consists of the following amino acid sequences:
(1) the amino acid sequence set forth in SEQ ID NO: 16, or an amino acid sequence having at least 90%, 91%, 92%, 93%, 94%, 95%, 96%, 97%, 98%, or 99% sequence identity to the amino acid sequence set forth in SEQ ID NO: 16 and having a substitution to cysteine at position 160, or an amino acid sequence having at least 90%, 91%, 92%, 93%, 94%, 95%, 96%, 97%, 98%, or 99% sequence identity to the amino acid sequence set forth in SEQ ID NO: 16 and comprising the amino acid sequence set forth in SEQ ID NO: 7;
(2) the amino acid sequence set forth in SEQ ID NO: 17, or an amino acid sequence having at least 90%, 91%, 92%, 93%, 94%, 95%, 96%, 97%, 98%, or 99% sequence identity to the amino acid sequence set forth in SEQ ID NO: 17 and having a substitution to cysteine at position 166, or an amino acid sequence having at least 90%, 91%, 92%, 93%, 94%, 95%, 96%, 97%, 98%, or 99% sequence identity to the amino acid sequence set forth in SEQ ID NO: 17 and comprising the amino acid sequence set forth in SEQ ID NO: 8;
(3) the amino acid sequence set forth in SEQ ID NO: 18, or an amino acid sequence having at least 90%, 91%, 92%, 93%, 94%, 95%, 96%, 97%, 98%, or 99% sequence identity to the amino acid sequence set forth in SEQ ID NO: 18 and having substitutions to cysteine at positions 160 and 166, or an amino acid sequence having at least 90%, 91%, 92%, 93%, 94%, 95%, 96%, 97%, 98%, or 99% sequence identity to the amino acid sequence set forth in SEQ ID NO: 18 and comprising the amino acid sequence set forth in SEQ ID NO: 28;
(4) the amino acid sequence set forth in SEQ ID NO: 19, or an amino acid sequence having at least 90%, 91%, 92%, 93%, 94%, 95%, 96%, 97%, 98%, or 99% sequence identity to the amino acid sequence set forth in SEQ ID NO: 19 and having a substitution to cysteine at position 138, or an amino acid sequence having at least 90%, 91%, 92%, 93%, 94%, 95%, 96%, 97%, 98%, or 99% sequence identity to the amino acid sequence set forth in SEQ ID NO: 19 and comprising the amino acid sequence set forth in SEQ ID NO: 12;
(5) the amino acid sequence set forth in SEQ ID NO: 20, or an amino acid sequence having at least 90%, 91%, 92%, 93%, 94%, 95%, 96%, 97%, 98%, or 99% sequence identity to the amino acid sequence set forth in SEQ ID NO: 20 and having a substitution to cysteine at position 165, or an amino acid sequence having at least 90%, 91%, 92%, 93%, 94%, 95%, 96%, 97%, 98%, or 99% sequence identity to the amino acid sequence set forth in SEQ ID NO: 20 and comprising the amino acid sequence set forth in SEQ ID NO: 9;
(6) the amino acid sequence set forth in SEQ ID NO: 21, or an amino acid sequence having at least 90%, 91%, 92%, 93%, 94%, 95%, 96%, 97%, 98%, or 99% sequence identity to the amino acid sequence set forth in SEQ ID NO: 21 and having a substitution to cysteine at position 167, or an amino acid sequence having at least 90%, 91%, 92%, 93%, 94%, 95%, 96%, 97%, 98%, or 99% sequence identity to the amino acid sequence set forth in SEQ ID NO: 21 and comprising the amino acid sequence set forth in SEQ ID NO: 14; or
(7) the amino acid sequence set forth in SEQ ID NO: 60, or an amino acid sequence having at least 90%, 91%, 92%, 93%, 94%, 95%, 96%, 97%, 98%, or 99% sequence identity to the amino acid sequence set forth in SEQ ID NO: 60 and having substitutions to cysteine at positions 160 and 166 and having a substitution to tyrosine at position 178, or an amino acid sequence having at least 90%, 91%, 92%, 93%, 94%, 95%, 96%, 97%, 98%, or 99% sequence identity to the amino acid sequence set forth in SEQ ID NO: 60 and comprising the amino acid sequence set forth in SEQ ID NO: 62.

In some embodiments, the modified CH1 of the present invention is CH1 of IgG1, and comprises the amino acid sequence set forth in SEQ ID NO: 10 or 11.

In some embodiments, the modified CH1 of the present invention comprises or consists of the following amino acid sequences:
(1) the amino acid sequence set forth in SEQ ID NO: 22, or an amino acid sequence having at least 90%, 91%, 92%, 93%, 94%, 95%, 96%, 97%, 98%, or 99% sequence identity to the amino acid sequence set forth inSEQ ID NO: 22 and having a substitution to cysteine at position 152, or an amino acid sequence having at least 90%, 91%, 92%, 93%, 94%, 95%, 96%, 97%, 98%, or 99% sequence identity to the amino acid sequence set forth in SEQ ID NO: 22 and comprising the amino acid sequence set forth in SEQ ID NO: 10; or
(2) the amino acid sequence set forth in SEQ ID NO: 23, or an amino acid sequence having at least 90%, 91%, 92%, 93%, 94%, 95%, 96%, 97%, 98%, or 99% sequence identity to the amino acid sequence set forth in SEQ ID NO: 23 and having a substitution to cysteine at position 155, or an amino acid sequence having at least 90%, 91%, 92%, 93%, 94%, 95%, 96%, 97%, 98%, or 99% sequence identity to the amino acid sequence set forth in SEQ ID NO: 23 and comprising the amino acid sequence set forth in SEQ ID NO: 11.

In some embodiments, the modified Fc region of the present invention is the Fc region of IgG1 and comprises the amino acid sequence set forth in SEQ ID NO: 13, or comprises the amino acid sequences set forth in SEQ ID NOs: 67 and 68.

In some embodiments, the modified Fc region of the present invention comprises or consists of the following amino acid sequences:
the amino acid sequence set forth in SEQ ID NO: 24, or an amino acid sequence having at least 90%, 91%, 92%, 93%, 94%, 95%, 96%, 97%, 98%, or 99% sequence identity to the amino acid sequence set forth in SEQ ID NO: 24 and having a substitution to cysteine at position 290, or an amino acid sequence having at least 90%, 91%, 92%, 93%, 94%, 95%, 96%, 97%, 98%, or 99% sequence identity to the amino acid sequence set forth in SEQ ID NO: 24 and comprising the amino acid sequence as shown in SEQ ID NO: 13; or
the amino acid sequence set forth in SEQ ID NO: 65, or an amino acid sequence having at least 90%, 91%, 92%, 93%, 94%, 95%, 96%, 97%, 98%, or 99% sequence identity to the amino acid sequence set forth in SEQ ID NO: 65 and having substitutions to cysteine at positions 239 and 375, or an amino acid sequence having at least 90%, 91%, 92%, 93%, 94%, 95%, 96%, 97%, 98%, or 99% sequence identity to the amino acid sequence set forth in SEQ ID NO: 65 and comprising the amino acid sequence set forth in SEQ ID NO: 67 and SEQ ID NO: 68.

In some embodiments, the modified heavy chain constant region of the present invention is the heavy chain constant region of IgG 1, and comprises the amino acid sequence set forth in SEQ ID NO: 10, 11, or 13.

In some embodiments, the modified heavy chain constant region of the present invention
(1) comprises or consists of the amino acid sequence set forth in SEQ ID NO: 25, or comprises an amino acid sequence having at least 90%, 91%, 92%, 93%, 94%, 95%, 96%, 97%, 98%, or 99% sequence identity to the amino acid sequence set forth in SEQ ID NO: 25 and having a substitution to cysteine at position 152, or comprises an amino acid sequence having at least 90%, 91%, 92%, 93%, 94%, 95%, 96%, 97%, 98%, or 99% sequence identity to the amino acid sequence set forth in SEQ ID NO: 25 and comprising the amino acid sequence set forth in SEQ ID NO: 10;
(2) comprises or consists of the amino acid sequence set forth in SEQ ID NO: 26, or comprises an amino acid sequence having at least 90%, 91%, 92%, 93%, 94%, 95%, 96%, 97%, 98%, or 99% sequence identity to the amino acid sequence set forth in SEQ ID NO: 26 and having a substitution to cysteine at position 155, or comprises an amino acid sequence having at least 90%, 91%, 92%, 93%, 94%, 95%, 96%, 97%, 98%, or 99% sequence identity to the amino acid sequence set forth in SEQ ID NO: 26 and comprising the amino acid sequence set forth in SEQ ID NO: 11;
(3) comprises or consists of the amino acid sequence set forth in SEQ ID NO: 27, or comprises an amino acid sequence having at least 90%, 91%, 92%, 93%, 94%, 95%, 96%, 97%, 98%, or 99% sequence identity to the amino acid sequence set forth in SEQ ID NO: 27 and having a substitution to cysteine at position 290, or comprises an amino acid sequence having at least 90%, 91%, 92%, 93%, 94%, 95%, 96%, 97%, 98%, or 99% sequence identity to the amino acid sequence set forth in SEQ ID NO: 27 and comprising the amino acid sequence set forth in SEQ ID NO: 13; or
(4) comprises or consists of the amino acid sequence set forth in SEQ ID NO: 64, or comprises an amino acid sequence having at least 90%, 91%, 92%, 93%, 94%, 95%, 96%, 97%, 98%, or 99% sequence identity to the amino acid sequence set forth in SEQ ID NO: 64 and having substitutions to cysteine at positions 239 and 375, or comprises an amino acid sequence having at least 90%, 91%, 92%, 93%, 94%, 95%, 96%, 97%, 98%, or 99% sequence identity to the amino acid sequence set forth in SEQ ID NO: 64 and comprising the amino acid sequence set forth in SEQ ID NO: 67 and SEQ ID NO: 68.

In some embodiments, the modified antibody or antigen-binding fragment thereof of the present invention comprises a modified light chain constant region, wherein the light chain constant region
comprises or consists of the amino acid sequence set forth in SEQ ID NO: 18, or comprises an amino acid sequence having at least 90%, 91%, 92%, 93%, 94%, 95%, 96%, 97%, 98%, or 99% sequence identity to the amino acid sequence set forth in SEQ ID NO: 18 and having substitutions to cysteine at positions 160 and 166, or comprises an amino acid sequence having at least 90%, 91%, 92%, 93%, 94%, 95%, 96%, 97%, 98%, or 99% sequence identity to the amino acid sequence set forth in SEQ ID NO: 18 and comprising the amino acid sequence set forth in SEQ ID NO: 28.

In some embodiments, the modified antibody or antigen-binding fragment thereof of the present invention comprises a modified light chain constant region and a heavy chain constant region, wherein the modified light chain constant region
comprises or consists of the amino acid sequence set forth in SEQ ID NO: 18, or comprises an amino acid sequence having at least 90%, 91%, 92%, 93%, 94%, 95%, 96%, 97%, 98%, or 99% sequence identity to the amino acid sequence set forth in SEQ ID NO: 18 and having substitutions to cysteine at positions 160 and 166, or comprises an amino acid sequence having at least 90%, 91%, 92%, 93%, 94%, 95%, 96%, 97%, 98%, or 99% sequence identity to the amino acid sequence set forth in SEQ ID NO: 18 and comprising the amino acid sequence set forth in SEQ ID NO: 28; and
the heavy chain constant region comprises a human IgG1 heavy chain constant region, for example
   comprises or consists of the amino acid sequence set forth in SEQ ID NO: 3, or comprises an amino acid sequence having at least 90%, 91%, 92%, 93%, 94%, 95%, 96%, 97%, 98%, or 99% sequence identity to the amino acid sequence set forth in SEQ ID NO: 3 and having no cysteine substitution.

In some embodiments, the modified antibody or antigen-binding fragment thereof of the present invention comprises a modified light chain constant region, and a heavy chain constant region, wherein the modified light chain constant region
comprises or consists of the amino acid sequence set forth in SEQ ID NO: 60, or comprises an amino acid sequence having at least 90%, 91%, 92%, 93%, 94%, 95%, 96%, 97%, 98%, or 99% sequence identity to the amino acid sequence set forth in SEQ ID NO: 60 and having substitutions to cysteine at positions 160 and 166 and having a substitution to tyrosine at position 178, or comprises an amino acid sequence having at least 90%, 91%, 92%, 93%, 94%, 95%, 96%, 97%, 98%, or 99% sequence identity to the amino acid sequence set forth in SEQ ID NO: 60 and comprising the amino acid sequence set forth in SEQ ID NO: 62, and
the heavy chain constant region comprises a human IgG1 heavy chain constant region, for example
   comprises or consists of the amino acid sequence set forth in SEQ ID NO: 3, or comprises an amino acid sequence having at least 90%, 91%, 92%, 93%, 94%, 95%, 96%, 97%, 98%, or 99% sequence identity to the amino acid sequence set forth in SEQ ID NO:3 and having no cysteine substitution.

In some embodiments, the modified antibody or antigen-binding fragment thereof of the present invention comprises a modified light chain constant region and a modified heavy chain constant region, wherein
the modified light chain constant region
comprises or consists of the amino acid sequence set forth in SEQ ID NO: 17, or comprises an amino acid sequence having at least 90%, 91%, 92%, 93%, 94%, 95%, 96%, 97%, 98%, or 99% sequence identity to the amino acid sequence set forth in SEQ ID NO: 17 and having a substitution to cysteine at position 166, or comprises an amino acid sequence having at least 90%, 91%, 92%, 93%, 94%, 95%, 96%, 97%, 98%, or 99% sequence identity to the amino acid sequence set forth in SEQ ID NO: 17 and comprising the amino acid sequence set forth in SEQ ID NO: 8;
and the modified heavy chain constant region
   (1) comprises or consists of the amino acid sequence set forth in SEQ ID NO: 25, or comprises an amino acid sequence having at least 90%, 91%, 92%, 93%, 94%, 95%, 96%, 97%, 98%, or 99% sequence identity to the amino acid sequence set forth in SEQ ID NO: 25 and having a substitution to cysteine at position 152, or comprises an amino acid sequence having at least 90%, 91%, 92%, 93%, 94%, 95%, 96%, 97%, 98%, or comprises 99% sequence identity to the amino acid sequence set forth in SEQ ID NO: 25 and comprising the amino acid sequence set forth in SEQ ID NO: 10; or
   (2) comprises or consists of the amino acid sequence set forth in SEQ ID NO: 26, or comprises an amino acid sequence having at least 90%, 91%, 92%, 93%, 94%, 95%, 96%, 97%, 98%, or 99% sequence identity to the amino acid sequence set forth in SEQ ID NO: 26 and having a substitution to cysteine at position 155, or comprises an amino acid sequence having at least 90%, 91%, 92%, 93%, 94%, 95%, 96%, 97%, 98%, or 99% sequence identity to the amino acid sequence set forth in SEQ ID NO: 26 and comprising the amino acid sequence set forth in SEQ ID NO: 11; or
   (3) comprises a modified CH1, which
      (i) comprises or consistis of the amino acid sequence set forth in SEQ ID NO: 22, or comprises an amino acid sequence having at least 90%, 91%, 92%, 93%, 94%, 95%, 96%, 97%, 98%, or 99% sequence identity to the amino acid sequence set forth in SEQ ID NO: 22 and having a substitution to cysteine at position 152, or comprises an amino acid sequence having at least 90%, 91%, 92%, 93%, 94%, 95%, 96%, 97%, 98%, or 99% sequence identity to the amino acid sequence set forth in SEQ ID NO: 22 and comprising the amino acid sequence set forth in SEQ ID NO: 10; or
      (ii) comprises or consists of the amino acid sequence set forth in SEQ ID NO: 23, or comprises an amino acid sequence having at least 90%, 91%, 92%, 93%, 94%, 95%, 96%, 97%, 98%, or 99% sequence identity to the amino acid sequence set forth in SEQ ID NO: 23 and having a substitution to cysteine at position 155, or comprises an amino acid sequence having at least 90%, 91%, 92%, 93%, 94%, 95%, 96%, 97%, 98%, or 99% sequence identity to the amino acid sequence set forth in SEQ ID NO: 23 and comprising the amino acid sequence set forth in SEQ ID NO: 11.

In some embodiments, the modified antibody or antigen-binding fragment thereof of the present invention comprises a modified light chain constant region and a modified heavy chain constant region, wherein
the modified light chain constant region
comprises or consists of the amino acid sequence set forth in SEQ ID NO: 44, or comprises an amino acid sequence having at least 90%, 91%, 92%, 93%, 94%, 95%, 96%, 97%, 98%, or 99% sequence identity to the amino acid sequence set forth in SEQ ID NO: 44 and having a substitution to cysteine at position 166, or comprises an amino acid sequence having at least 90%, 91%, 92%, 93%, 94%, 95%, 96%, 97%, 98%, or 99% sequence identity to the amino acid sequence set forth in SEQ ID NO: 44 and comprising the amino acid sequence set forth in SEQ ID NO: 66;
and the modified heavy chain constant region
   (1) comprises or consists of the amino acid sequence set forth in SEQ ID NO: 61, or comprises an amino acid sequence having at least 90%, 91%, 92%, 93%, 94%, 95%, 96%, 97%, 98%, or 99% sequence identity to the amino acid sequence set forth in SEQ ID NO: 61 and having a substitution to cysteine at position 375, or comprises an amino acid sequence having at least 90%, 91%, 92%, 93%, 94%, 95%, 96%, 97%, 98%, or 99% sequence identity to the amino acid sequence set forth in SEQ ID NO: 61 and comprising the amino acid sequence as shown in SEQ ID NO: 67; or
   (2) comprises a modified Fc region which comprises or consists of the amino acid sequence set forth in SEQ ID NO: 63, or comprises an amino acid sequence having at least 90%, 91%, 92%, 93%, 94%, 95%, 96%, 97%, 98%, or 99% sequence identity to the amino acid sequence set forth in SEQ ID NO: 63 and having a substitution to cysteine at position 375, or comprises an amino acid sequence having at least 90%, 91%, 92%, 93%, 94%, 95%, 96%, 97%, 98%, or 99% sequence identity to the amino acid sequence set forth in SEQ ID NO: 63 and comprising the amino acid sequence set forth in SEQ ID NO: 67.

In some embodiments, the modified antibody or antigen-binding fragment thereof of the present invention comprises a modified heavy chain constant region and a modified light chain constant region, wherein
the modified heavy chain constant region
   (1) comprises or consists of the amino acid sequence set forth in SEQ ID NO: 64, or comprises an amino acid sequence having at least 90%, 91%, 92%, 93%, 94%, 95%, 96%, 97%, 98%, or 99% sequence identity to the amino acid sequence set forth in SEQ ID NO: 64 and having substitutions to cysteine at positions 239 and 375, or comprises an amino acid sequence having at least 90%, 91%, 92%, 93%, 94%, 95%, 96%, 97%, 98%, or 99% sequence identity to the amino acid sequence set forth in SEQ ID NO: 64 and comprising the amino acid sequence set forth in SEQ ID NO:67 and SEQ ID NO: 68; or
   (2) comprises a modified Fc region which comprises or consists of of the amino acid sequence set forth in SEQ ID NO: 65, or comprises an amino acid sequence having at least 90%, 91%, 92%, 93%, 94%, 95%, 96%, 97%, 98%, or 99% sequence identity to the amino acid sequence set forth in SEQ ID NO: 65 and having a substitution to cysteine at position 375, or comprises an amino acid sequence having at least 90%, 91%, 92%, 93%, 94%, 95%, 96%, 97%, 98%, or 99% sequence identity to the amino acid sequence set forth in SEQ ID NO: 65 and comprising the amino acid sequence set forth in SEQ ID NO: 67 and SEQ ID NO: 68;
and the light chain constant region comprises a Kappa light chain constant region or a Lambda light chain constant region, which, for example,
comprises or consists of the amino acid sequence set forth in SEQ ID NO: 5 or 6, or comprises an amino acid sequence having at least 90%, 91%, 92%, 93%, 94%, 95%, 96%, 97%, 98%, or 99% sequence identity to the amino acid sequence set forth in SEQ ID NO: 5 or 6 and having no cysteine substitution.

In some embodiments, the modified antibody or antigen-binding fragment thereof of the present invention comprises a modified light chain constant region and a modified heavy chain constant region, wherein
the modified light chain constant region
comprises or consists of the amino acid sequence set forth in SEQ ID NO: 20, or comprises an amino acid sequence having at least 90%, 91%, 92%, 93%, 94%, 95%, 96%, 97%, 98%, or 99% sequence identity to the amino acid sequence set forth in SEQ ID NO: 20 and having a substitution to cysteine at position 165, or comprises an amino acid sequence having at least 90%, 91%, 92%, 93%, 94%, 95%, 96%, 97%, 98%, or 99% sequence identity to the amino acid sequence set forth in SEQ ID NO: 20 and comprising the amino acid sequence set forth in SEQ ID NO: 9;
and the modified heavy chain constant region
   (1) comprises or consists of the amino acid sequence set forth in SEQ ID NO: 25, or comprises an amino acid sequence having at least 90%, 91%, 92%, 93%, 94%, 95%, 96%, 97%, 98%, or 99% sequence identity to the amino acid sequence set forth in SEQ ID NO: 25 and having a substitution to cysteine at position 152, or comprises an amino acid sequence having at least 90%, 91%, 92%, 93%, 94%, 95%, 96%, 97%, 98%, or 99% sequence identity to the amino acid sequence set forth in SEQ ID NO: 25 and comprising the amino acid sequence as shown in SEQ ID NO: 10; or
   (2) comprises or consists of the amino acid sequence set forth in SEQ ID NO: 26, or comprises an amino acid sequence having at least 90%, 91%, 92%, 93%, 94%, 95%, 96%, 97%, 98%, or 99% sequence identity to the amino acid sequence set forth in SEQ ID NO: 26 and having a substitution to cysteine at position 155, or comprises an amino acid sequence having at least 90%, 91%, 92%, 93%, 94%, 95%, 96%, 97%, 98%, or 99% sequence identity to the amino acid sequence set forth in SEQ ID NO: 26 and comprising the amino acid sequence as shown in SEQ ID NO: 11; or
   (3) comprises a modified CH1, which
      (i) comprises or consists of the amino acid sequence set forth in SEQ ID NO: 22, or comprises an amino acid sequence having at least 90%, 91%, 92%, 93%, 94%, 95%, 96%, 97%, 98%, or 99% sequence identity to the amino acid sequence set forth in SEQ ID NO: 22 and having a substitution to cysteine at position 152, or comprises an amino acid sequence having at least 90%, 91%, 92%, 93%, 94%, 95%, 96%, 97%, 98%, or 99% sequence identity to the amino acid sequence set forth in SEQ ID NO: 22 and comprising the amino acid sequence set forth in SEQ ID NO: 10; or
      (ii) comprises or consists of the amino acid sequence set forth in SEQ ID NO: 23, or comprises an amino acid sequence having at least 90%, 91%, 92%, 93%, 94%, 95%, 96%, 97%, 98%, or 99% sequence identity to the amino acid sequence set forth in SEQ ID NO: 23 and having a substitution to cysteine at position 155, or comprises an amino acid sequence having at least 90%, 91%, 92%, 93%, 94%, 95%, 96%, 97%, 98%, or 99% sequence identity to the amino acid sequence set forth in SEQ ID NO: 23 and comprising the amino acid sequence set forth in SEQ ID NO: 11.

In some embodiments, the modified antibody or antigen-binding fragment thereof of the present invention comprises a modified light chain constant region and a modified heavy chain constant region, wherein
the modified light chain constant region
comprises or consists of the amino acid sequence set forth in SEQ ID NO: 16, or comprises an amino acid sequence having at least 90%, 91%, 92%, 93%, 94%, 95%, 96%, 97%, 98%, or 99% sequence identity to the amino acid sequence set forth in SEQ ID NO: 16 and having a substitution to cysteine at position 160, or comprises an amino acid sequence having at least 90%, 91%, 92%, 93%, 94%, 95%, 96%, 97%, 98%, or 99% sequence identity to the amino acid sequence set forth in SEQ ID NO: 16 and comprising the amino acid sequence set forth in SEQ ID NO: 7;
and the modified heavy chain constant region
   (1) comprises or consists of the amino acid sequence set forth in SEQ ID NO: 25, or comprises an amino acid sequence having at least 90%, 91%, 92%, 93%, 94%, 95%, 96%, 97%, 98%, or 99% sequence identity to the amino acid sequence set forth in SEQ ID NO: 25 and having a substitution to cysteine at position 152, or comprises an amino acid sequence having at least 90%, 91%, 92%, 93%, 94%, 95%, 96%, 97%, 98%, or 99% sequence identity to the amino acid sequence set forth in SEQ ID NO: 25 and comprising the amino acid sequence as shown in SEQ ID NO: 10; or
   (2) comprises or consists of the amino acid sequence set forth in SEQ ID NO: 26, or comprises an amino acid sequence having at least 90%, 91%, 92%, 93%, 94%, 95%, 96%, 97%, 98%, or 99% sequence identity to the amino acid sequence set forth in SEQ ID NO: 26 and having a substitution to cysteine at position 155, or comprises an amino acid sequence having at least 90%, 91%, 92%, 93%, 94%, 95%, 96%, 97%, 98%, or 99% sequence identity to the amino acid sequence set forth in SEQ ID NO: 26 and comprising the amino acid sequence as shown in SEQ ID NO: 11;
   (3) comprises a modified CH1 which
      (i) comprises or consists of the amino acid sequence set forth in SEQ ID NO: 22, or comprises an amino acid sequence having at least 90%, 91%, 92%, 93%, 94%, 95%, 96%, 97%, 98%, or 99% sequence identity to the amino acid sequence set forth in SEQ ID NO: 22 and having a substitution to cysteine at position 152, or comprises an amino acid sequence having at least 90%, 91%, 92%, 93%, 94%, 95%, 96%, 97%, 98%, or 99% sequence identity to the amino acid sequence set forth in SEQ ID NO: 22 and comprising the amino acid sequence set forth in SEQ ID NO: 10; or
      (ii) comprises or consists of the amino acid sequence set forth in SEQ ID NO: 23, or comprises an amino acid sequence having at least 90%, 91%, 92%, 93%, 94%, 95%, 96%, 97%, 98%, or 99% sequence identity to the amino acid sequence set forth in SEQ ID NO: 23 and having a substitution to cysteine at position 155, or comprises an amino acid sequence having at least 90%, 91%, 92%, 93%, 94%, 95%, 96%, 97%, 98%, or 99% sequence identity to the amino acid sequence set forth in SEQ ID NO: 23 and comprising the amino acid sequence set forth in SEQ ID NO: 11.

The modified antibody or antigen-binding fragment of the present invention can be in any form known in the art, such as monoclonal, chimeric, humanized, complete human, full-length, bispecific, or multispecific antibody or antibody fragment thereof.

In some embodiments, the modified antibody of the present invention specifically binds to an antigen. In some embodiments, the antigen is a tumor-specific antigen (TA) or a tumor-associated antigen (TAA). In some embodiments, the tumor-associated antigen is an immune checkpoint molecule. In some embodiments, the antigen is selected from FAP, CEA, p95, HER2, BCMA, EpCAM, MSLN, MCSP, HER-1, HER-2, CD19, CD20, CD22, CD33, CD38, CD52, Flt3, EpCAM, IGF-1R, FOLR1, Trop-2, CA-12-5, HLA-DR, MUC-1 (mucin), GD2, A33-antigen, PSMA, PSCA, transferrin-receptor, TNC (tenascin), CA-IX, CD3, B7H3, Hel, cMET, Axl, GPRC5D, PD-1, PD-L1, CD47, immune-activating molecules such as 4-1BB, CD40, OX40, and the like. In some embodiments, the antigen is HER2.

In some embodiments, the parent antibody of the present invention is derived from an antibody that specifically binds to HER2, such as trastuzumab. In some embodiments, the parent antibody of the present invention comprises 1, 2, 3, 4, 5 or 6 CDRs of a known antibody that specifically binds to HER2, such as trastuzumab. In some embodiments, the parent antibody of the present invention comprises 1, 2, and 3 heavy chain variable region CDRs, i.e., HCDR1, HCDR2, and HCDR3, of a known antibody that specifically binds to Her2, such as trastuzumab. In some embodiments, the parent antibody of the present invention comprises 1, 2, and 3 light chain variable region CDRs, i.e., LCDR1, LCDR2, and LCDR3, of a known antibody that specifically binds HER2, such as trastuzumab. In some embodiments, the parent antibody of the present invention comprises 3 heavy chain variable region CDRs and 3 light chain variable region CDRs of a known antibody that specifically binds to HER2, such as trastuzumab. In some embodiments, the parent antibody of the present invention comprises the heavy chain variable region and the light chain variable region of a known antibody that specifically binds to HER2, such as trastuzumab.

In some embodiments, the modified antibody or antigen-binding fragment of the present invention is a full-length antibody comprising the modified heavy chain constant region, or the modified CH1, or the modified Fc region as described herein. In some embodiments, the modified antibody or antigen-binding fragment of the present invention is a full-length antibody comprising the modified light chain constant region described herein. In some embodiments, the modified antibody or antigen-binding fragment of the present invention is a full-length antibody comprising the modified light chain constant region described herein, and the modified heavy chain constant region described herein. In some embodiments, the modified antibody or antigen-binding fragment comprises two heavy chains and two light chains, wherein one or both of the two heavy chains respectively comprise the heavy chain constant region mutation or the combination of mutations described herein, or one or both of the two light chains respectively comprise the light chain constant region mutation or the combination of mutations described herein; or both the two heavy chains and both the two light chains comprise the heavy chain constant region mutation or the combination of mutations, and the light chain constant region mutation combination as described herein.

In some embodiments, the modified antibody of the present invention is a bispecific antibody comprising a first antigen-binding region that specifically binds to a first antigen, and a second antigen-binding region that specifically binds to a second antigen. In some embodiments, the bispecific antibody comprises:
a first heavy chain: VH_{Ab1}-a first heavy chain constant region;
a first light chain: VL_{Ab1}-a first light chain constant region;
a second heavy chain: VH_{Ab2}-a second heavy chain constant region; and
a second light chain: VL_{Ab2}-a second light chain constant region;
wherein the first and second light chain constant regions are the modified light chain constant regions as defined herein; and/or the first and the second heavy chain constant region are the modified heavy chain constant regions described herein;
VH_{Ab1} and VL_{Ab1} are the heavy chain variable region VH and light chain variable region VL of the antigen-binding region that specifically binds to the first antigen respectively, and VH_{Ab2} and VL_{Ab2} are the heavy chain variable region VH and light chain variable region VL of the antigen-binding region that specifically binds to the second antigen, respectively.

In some embodiments, the first heavy chain constant region and the second heavy chain constant region are identical, or have the same mutaion(s) to cysteine. In some embodiments, the first light chain constant region and the second light chain constant region are identical, or have the same mutaion(s) to cysteine.

In some embodiments, the first heavy chain constant region and the second heavy chain constant region do not have a mutation to cysteine of the present invention and the first light chain constant region and/or the second light chain constant region have mutation(s) to cysteine of the present invention (and optionally other mutation(s) such as the 178Y mutation), preferably the first light chain constant region and the second light chain constant region each has the same mutation(s) to cysteine of the present invention, more preferably the first light chain constant region and the second light chain constant region are identical and each has mutation(s) to cysteine of the present invention (and optionally other mutation(s) such as the 178Y mutation). In some embodiments, the first heavy chain constant region and the second heavy chain constant region each is the heavy chain constant region without a mutation to cysteine of the present invention, and the first light chain constant region and the second light chain constant region each is a Lambda light chain constant region with mutations to cysteine of the present invention at positions 160 and 166. In some embodiments, the first heavy chain constant region and the second heavy chain constant region each is a heavy chain constant region without a mutation to cysteine of the present invention, and the first light chain constant region and the second light chain constant region each is a Kappa light chain constant region with mutations to cysteine of the present invention at positions 160 and 166, and a mutation to tyrosine at position 178.

In some embodiments, the first heavy chain constant region and the second heavy chain constant region each has mutation(s) to cysteine of the present invention, e.g., has the same mutation(s) to cysteine of the present invention, and the first light chain constant region and the second light chain constant region each does not have a mutation to cysteine of the present invention.

In some embodiments, the first heavy chain constant region and the first light chain constant region each has mutation(s) to cysteine of the present invention, e.g., the first heavy chain constant region and the first light chain constant region each has mutation(s) to cysteine described herein, e.g., each is the modified heavy chain constant region and light chain constant region of the combination of modified heavy chain constant region and light chain constant region as defined herein above, and/or the second heavy chain constant region and the second light chain constant region eac has mutation(s) to cysteine of the present invention, e.g., the second heavy chain constant region and the second light chain constant region each has mutation(s) to cysteine described herein, e.g., each is the modified heavy chain constant region and light chain constant region of the combination of modified heavy chain constant region and light chain constant region as defined herein above. In some embodiments, the first heavy chain constant region and the second heavy chain constant region each has the same mutation(s) to cysteine, and the first light chain constant region and the second light chain constant region each has the same mutation(s) to cysteine.

In some embodiments, the first heavy chain constant region and the second heavy chain constant regions further comprise a first Fc region and a second Fc region, respectively, and the first Fc region and the second Fc region comprises mutations facilitating heterodimerization thereof respectively. Methods for promoting heterodimerization of Fc regions are known in the art. For example, the CH3 region of the first Fc region and the CH3 region of the second Fc region are engineered in a complementary manner such that each CH3 region (or the heavy chain comprising the same) is no longer able to homodimerize with itself but is forced to be heterodimerized with other CH3 regions that are complementarily engineered (such that the CH3 regions of the first and second Fc regions are heterodimerized and no homodimers are formed between the two first CH3 regions or the two second CH3 regions). For example, the respective Knob mutations and Hole mutations are introduced in the first Fc region and the second Fc region based on the Knob-in-Hole technology. See, e.g., US 5, 731, 168; US 7, 695, 936; Ridgway et al., Prot Eng 9, 617-621(1996) and Carter, J Immunol Meth 248 ,7-15(2001) for this technology. Corresponding mutations may also be introduced in the first CH3 region of the first Fc region and the second CH3 region of the second Fc region based on the Innobody technology. See, for example, WO2022143912A1 (which is incorporated herein in its entirety) for this technology.

In some embodiments, the first antigen and the second antigen are tumor-specific antibodies (TAs) or tumor-associated antigens (TAAs). In some embodiments, the tumor-associated antigen is an immune checkpoint molecule. In some embodiments, each of the antigens is selected from FAP, CEA, p95, HER2, BCMA, EpCAM, MSLN, MCSP, HER-1, HER-2, CD19, CD20, CD22, CD33, CD38, CD52Flt3, EpCAM, IGF-1R, FOLR1, Trop-2, CA-12-5, HLA-DR, MUC-1(mucin), GD2, A33-antigen, PSMA, PSCA, transferrin-receptor, TNC(tenascin), CA-IX, CD3, B7H3, Hel, cMET, Axl, GPRC5D, PD-1, PD-L1, CD47, immune-activating molecules such as 4-1BB, CD40, OX40, and the like. In some embodiments, the first antigen and the second antigen are HER3 and EGFR, respectively.

In some embodiments, the bispecific antibody that specifically binds to HER3 and EGFR constructed by comprising the combination of LLC160-LLC166 mutation sites of the present invention exhibits superior properties in terms of antibody expression yield, one-step purification efficiency, DAR value and SEC purity (aggregation) of the ADC molecules after conjugation. The combination of LLC160-LLC166 mutation sites exhibits superior conjugation superiority relative to other mutation combinations.

In some embodiments, the bispecific antibody ADC molecule that specifically binds to HER3 and EGFR constructed by comprising the combination of LLC 160-LLC 166 mutation sites of the present invention has one or more of the following properties:
(1) In a plasma stability test, the bsAb-ADC molecule is very stable in plasma, e.g., mouse plasma or monkey plasma, and no significant drug shedding is observed;
(2) In an in vitro cell killing assay, it has significantly higher killing effect on tumor cells with lower EGFR expression and higher HER3 expression, such as human breast cancer cells MDA-MB-453, as compared to the other ADC constructed by bispecific antibodies without the mutation;
(3) It has strong affinity to both EGFR and HER3, and the site mutations do not affecting the binding activity of the antibody;
(4) It has anti-tumor effect and/or less side effect, for example, with insignificant effects on the body weight, for example, it has obvious inhibiting effect on the tumor in an H508 tumor-bearing mouse model or an NUGC-4 tumor-bearing mouse model or an SW620 tumor-bearing mouse model or an ASPC1 tumor-bearing mouse model or an NCI-H1568 tumor-bearing mouse model, without affecting the body weight of the mouse significantly.

### III. Preparation of cysteine-engineered antibody, encoding nucleic acid, expression vector and host cell

In one aspect of the present invention, the present invention relates to a method of preparing a cysteine-engineered antibody or antigen-binding fragment thereof comprising:
(a) substituting with cysteine at one or more amino acid positions (preferably at more cryptic sites) in the light chain constant region and/or heavy chain constant region (or CH1 or Fc region) of the antibody or antigen-binding fragment thereof;
(b) introducing a nucleic acid encoding each chain of the antibody or antigen-binding fragment thereof obtained in (a) or an expression vector comprising said nucleic acid into a host cell;
(c) expressing and assembling the antibody or antigen-binding fragment thereof in host cells;
optionally, the antibody or antigen-binding fragment thereof is purified, e.g., by Protein A.

In some embodiments, more cryptic sites are selected by analysis of the protein crystal structure of the antibody. In some specific embodiments, the selection includes the following steps:
resolving the crystal structure of the protein,
selecting one or more sites inside the Fab cavity formed by protein CH1 and VH, CL and VL, and inside the cavity formed by the two CH2 and two CH3 domains in the Fc region;
selecting a site inside the selected cavity as a candidate for a cryptic site;
determining the properties of the antibodies with mutations to cysteine at these cryptic sites or the immunoconjugates prepared with the antibodies.

In some embodiments, the cysteine-engineered antibody or antigen-binding fragment thereof of the present invention has or substantially maintains the expression level and affinity of the parent antibody or antigen-binding fragment thereof before mutation.

The present invention provides a nucleic acid encoding any chain or any monomer or domain of the antibody or antigen-binding fragment thereof of the present invention. Polynucleotide sequences encoding each chain can be generated using methods well known in the art. In addition, the polynucleotides and nucleic acids of the present invention may contain a segment encoding a secretory signal peptide which is operably linked to a nucleic acid encoding the antibody or antigen-binding fragment thereof of the present invention, thereby directs the secretory expression of the antibody or antigen-binding fragment thereof or individual chains thereof of the present invention.

The present invention also provides vectors comprising the nucleic acid of the present invention. In an embodiment, the vector is an expression vector, such as a eukaryotic expression vector. The vector includes, but are not limited to, viruses, plasmids, cosmids, λphages or yeast YACs. In a perferred embodiment, the expression vector of the present invention is a pcDNA vector, such as a pcDNA3.1 expression vector.

The present invention also provides a host cell comprising the nucleic acid or the vector. Host cells suitable for replicating and supporting the expression of the antibodies or antigen-binding fragments or individual chains thereof of the present invention are well known in the art. Such cells can be transfected or transduced with specific expression vectors, and large numbers of vector-containing cells can be grown for inoculation of large-scale fermenters, thereby obtaining sufficient quantities of multispecific antibodies, such as bispecific antibodies, for clinical use.

In an embodiment, the host cell is a eukaryotic cell. In another embodiment, the host cell is selected from a yeast cell, a mammalian cell, preferably a mammalian cell such as a Chinese hamster ovary (CHO) cell, a human embryonic kidney (HEK) cell or a lymphocytes (e.g., Y0, NS0, Sp20 cell) (e.g., a CHO cell or a 293 cell, such as an Expi293 cell, such as an Expi293F cell).

### IV. Immunoconjugate

In one aspect, the present invention also relates to an immunoconjugate comprising a modified antibody or antigen-binding fragment thereof, wherein the modified antibody or antigen-binding fragment thereof is defined herein.

By "immunoconjugate" is meant herein that the payload is attached to the antibody or antigen-binding fragment thereof by a linker so that the antibody or antigen-binding fragment thereof can act as a carrier to transport the payload by targeting to a target site.

The term "payload" refers to an active moiety conjugated to the antibody or antibody fragment of the present invention, and may include any moiety used to attach an antibody or antibody fragment. In some embodiments, the payload can be a drug, such as a small molecule drug, a radionuclide, DNA, RNA, an enzyme, or a polypeptide.

In some embodiments, the immunoconjugate encompasses an Antibody-Drug Conjugate (ADC), an Immunostimulatory Antibody Conjugate (ISAC), an Antibody-Oligonucleotide Conjugate (AOC), an Antibody-Polypeptide Conjugate (APC), an antibody Radionuclide Drug Conjugate (RDC), or an Antibody Degraducer Conjugate (ADeC).

Suitable payloads or active moieties for conjugation to the antibody include, for example, a cytotoxic agent, a chemotherapeutic agent, an innate immune agonist (e.g., Toll-like receptor agonist (TLR) ISAC drugs SBT6050, SBT6290, BDC-1001; STING agonist ISAC drug XMT-2056, Treg cell regulatory ISAC drug ADCT-301, etc.), an immunomodulator, a therapeutic oligonucleotide (siRNA, PMO, etc.), or a radionuclide, etc.

In some embodiments, the immunoconjugate of the present invention is an Antibody-Drug Conjugate namely ADC. In some embodiments, in the Antibody-Drug Conjugate, the drug moiety as a payload is selected from topoisomerase I inhibitors, e.g., camptothecin derivatives or camptothecins (e.g., DXd). In some embodiments, the drug moiety may be an anti-tubulin agent, such as auristatins (MMAF, MMAE), taxane analogs (e.g., epothilones A and B), maytansinoids (DM1, DM4), tubulysin, and analogs thereof. In some embodiments, the drug moiety may be a DNA acting drug, including PBD and the like.

In some embodiments, the DAR value of the immunoconjugate of the present invention is 1-6, e.g., 2, 3, 4, 5 or 6. In a preferred embodiment, the DAR value is 2 or 4.

In some embodiments, the average DAR value of the immunoconjugate of the present invention is 1-4, e.g., 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4.0, 4.1, 4.2, 4.3, or 4.4, and ranges having two of these values being endpoints.

In some embodiments, the payload (e.g., drug) of the immunoconjugate of the present invention is attached to the modified antibody or antibody fragment through a thiol group of the free cysteine(s) of the antibody or antigen-binding fragment thereof (optionally via a linker). In some embodiments, the payload (e.g., drug) of the immunoconjugate of the present invention is linked to a thiol group of the cysteine(s) through a cleavable or non-cleavable linker.

As used herein, "cleavable" refers to a linker that connects two moieties by covalent linkage, but is broken down under physiological conditions to separate the covalent linkage between the moieties. Cleavage may be enzymatic or non-enzymatic, but the payload is typically released from an antibody without degrading the antibody. As used herein, "non-cleavable" refers to a linker that is not susceptible to the breaking down under physiological conditions. While the linker may be physiologically modified, it remains the conjugation of the payload to the antibody until the antibody is substantially degraded, i.e., antibody degradation in vivo precedes the linker cleavage.

In one embodiment, the linker has a functional group capable of reacting with a thiol group on a cysteine residue present on the antibody or antigen-binding fragment thereof to form a covalent bond linking to the antibody or antigen-binding fragment thereof. Such non-limiting exemplary reactive functional groups are, for example, groups selected from the following groups: maleimide, iodoacetamide, bromoacetamide, vinylpyridine, disulfide, pyridyl disulfide, haloacetamide, α -haloacetyl, active esters such as succinimidyl esters, 4-nitrophenyl esters, pentafluorophenyl esters, tetrafluorophenyl esters, anhydrides, acyl chlorides, sulfonyl chlorides, isocyanates and isothiocyanates, preferably maleimide.

The linker may be made up of one or more linker components. Exemplary linker components include 6-maleimidocaproyl ("MC"), maleimidopropinoyl ("MP"), valine-citrulline ("val-cit" or "vc"), caproyl-glycine-glycine-phenylalanine-glycine (GGFG), alanine-phenylalanine ("ala-phe" or "af"), p-aminobenzyloxycarbonyl ("PAB"), N-succinimidyl 4- (2-pyridylthio) pentanoate ("SPP"), N-succinimidyl 4-(N-maleimidomethyl) cyclohexane-1 carboxylate ("SMCC"), and N-succinimidyl (4-iodo-acetyl) aminobenzoate ("SIAB"), ethyleneoxy-CH₂CH₂O-as one or more repeating units ("EO" or "PEO" ).

Exemplary linkers include, but are not limited to, a maleimido-caproyl-valine-alaine (mc-va) linker, a maleimidobutanoic acid-valine-citrulline (mb-vc) linker, or a maleimido-caproyl-glycine-glycine-phenylalanine-glycine linker (mc-GGFG).

In some embodiments, the immunoconjugates of the present invention comprise thiol-maleimides, haloacetyl groups, isothiocyanates, and the like.

In some embodiments, the payload in the immunoconjugate of the present invention may also be present in the form of a pharmaceutically acceptable salt.

In some embodiments, the payload in the immunoconjugate of the present invention may also be formed with a solvent molecule.

### V. Product and use

In some embodiments, the present invention also relates to a composition (e.g., a pharmaceutical composition or a pharmaceutical formulation) comprising the antibody or antigen-binding fragment thereof of the present invention or the immunoconjugate of the present invention.

In an embodiment, the composition further comprises a pharmaceutically acceptable excipient. In one embodiment, a composition, e.g., a pharmaceutical composition, comprises a binding molecule of the present invention in combination with one or more other therapeutic agents.

The composition of the present invention also contains suitable pharmaceutically acceptable excipient(s) such as pharmaceutical carriers, pharmaceutical vehicles, including buffers, as known in the art. As used herein, "pharmaceutically acceptable carrier" includes any and all solvents, dispersion media, isotonic agent or absorption delaying agents, and the like that are physiologically compatible. See also "Handbook of Pharmaceutical Excipients", 8th Edition, R.C. Rowe, P.J. Seskey and S.C. Owen, Pharmaceutical Press, London, Chicago for the use of pharmaceutically acceptable excipients and uses thereof. The compositions of the present invention can be in a variety of forms. Such forms include, for example, liquid, semi-solid, and solid dosage forms, such as liquid solutions (e.g., injectable solutions and infusible solutions), powders or suspensions, liposomal formulations, and suppositories. The preferred form depends on the intended mode of administration and therapeutic use. Medicaments comprising the antibody or immunoconjugate of the present invention described herein can be prepared by mixing the binding molecules of the present invention having the desired purity with one or more optional pharmaceutical excipients, for example in the form of lyophilized formulations or aqueous solutions.

In some embodiments, the present invention relates to the antibody or antigen-binding fragment thereof of the present invention or the immunoconjugate of the present invention for use in therapy, e.g., for the treatment of a tumor, e.g., cancer.

In some embodiments, the present invention relates to a method for treating a disease, e.g., a tumor such as cancer, using the antibody or antigen-binding fragment thereof of the present invention or the immunoconjugate of the present invention, or the use for the treatment, or the use for the manufacture of a medicament for the treatment.

In some embodiments, the tumor is a solid tumor or a hematological tumor. In some embodiments, the tumor is a HER2-positive tumor or cancer. In some embodiments, the tumor is breast cancer, such as HER2-positive breast cancer. In some embodiments, the tumor is a gastric cancer, such as HER2-positive gastric cancer.

Depending on its therapeutic use, the antibody or antigen-binding fragment thereof of the present invention or the immunoconjugate of the present invention may also be combined with other therapeutic agents. For example, when the antibody or antigen-binding fragment thereof or the immunoconjugate is used to treat a tumor, the therapeutic agent is, for example, a variety of therapeutic agents for treating a tumor, such as chemotherapeutic agents, angiogenesis inhibitors, cytokines, cytotoxic agents, other antibodies, small molecule drugs, or immunomodulatory agents (e.g., immune checkpoint inhibitors or agonists).

In some embodiments, the present invention also provides a pharmaceutical combination or a pharmaceutical combination product comprising the antibody or antigen-binding fragment thereof or the immunoconjugate of the present invention, and one or more other therapeutic agents, and the like.

A further object of the present invention is to provide a kit of parts comprising the pharmaceutical combination of the present invention, preferably in the form of drug dosage units. Dosage units may thus be provided according to a dosing regimen or interval of drug administration.

In an embodiment, a kit of parts according to the present invention comprises in the same package:
- a first container containing a pharmaceutical composition comprising the antibody or antigen-binding fragment thereof or an immunoconjugate of the present invention;
- a second container containing a pharmaceutical composition comprising another therapeutic agent.

### Examples

### Materials used in the Examples:

| | |
|---|---|
| Buffer 1 | 20 mM histidine buffer, pH 6.5 |
| Buffer 2 | 20 mM phosphate buffer, pH 7.0 |
| ADC Storage Buffer | 20 mM histidine buffer, pH 5.5 |
| HIC Mobile Phase A | 1.5 M ammonium sulfate, 50 mM sodium phosphate, pH 6.0 |
| HIC Mobile Phase B | 50 mM sodium phosphate, pH 6.0 / isopropanol (80:20 v/v) |
| SEC Buffer | 0.2 M PB, 0.25 M NaCl,15% (v/v) IPA, pH 7.0 |
| RP-HPLC Mobile Phase A | 0.1% TFA in ddH₂O |
| RP-HPLC Mobile Phase B | 0.1% TFA in ACN |
| FACS Buffer | PBS+2%FBS |

### Reagents and Columns

| Name | Manufacturer, Cat#, Lot# |
|---|---|
| Histidine | Sigma, Lot# SLCG1233 |
| TCEP | Aldrich, Lot# SLBZ2552 |
| DMSO | Sigma, Lot# RNBJ3655 |

| | |
|---|---|
| Na₂HPO₄ | SCR, Lot# 10039-32-4 |
| NaH₂PO₄ | SCR, Lot# 13472-35-0 |
| NaCl | SCR, Lot# 7647-14-5 |
| (NH₄)₂SO₄ | Aladdin, Lot# H2007071 |
| Isopropanol | Wochem, Lot# 67-63-0 |
| Desalting Column | Zeba^{™} Spin Desalting Columns, 40K MWCO, 2/5/10 mL |
| Amicon 30K | Amicon, Cat# UFC503096, Lot# R0PB38507 |
| SEC Column | TSK gel G3000SWXL, 7.8 mm × 30 cm, 5 µm |
| RP Column | Waters, BioResolve RP mAb Polyphenyl, 450 Å, 2.7 µm, 2.1 mm × 15 cm |
| HIC Column | TSKgel Butyl-NPR, 2.5 µm, 4.6 × 100 mm |
| 96-well plate | NUNC, 136101, 168607 |
| CellTiter-Glo^{®} Luminescent Cell Viabilit | PROMEGA, Cat#G7573, Lot#475537 |
| PBS | Gibco, Cat#10010-023, Lot#2339137 |
| FBS | Cytiva, Cat#SH30406.05 |

### Equipment

| Analysis | Equipment |
|---|---|
| RP-HPLC | Waters, ACQuity H class UPLC, SZ-A2-DD-HPLC01 |
| SEC Purity Analysis Equipment | Agilent, 1260, AS-146 |
| HIC Chromatographic Analysis Equipment | Agilent, 1260, AS-146 |
| CO2 Incubator | Thermo, 3111, SZ-A2-DD-IB07 |
| Microplate reader | MOLECULAR, SpetraMax i3x, SZ-A2-LAB01 |
| Centrifuge | Eppendorf, 5901R, SZ-A2-DD-CF06 |

### Example 1: Selection of protein mutation site

The present invention relates to a cysteine-engineered antibody molecule, with trastuzumab as a model antibody, wherein one or two amino acids are mutated to cysteines on its light chain or heavy chain, thus resulting in two or four cysteine-engineered antibody molecules due to the dimerization property of IgG antibodies. The thiol group on the cysteine can undergo a nucleophilic reaction with a toxin small molecule having a maleimide linker, thereby preparing a site-specifically conjugated ADC molecule by conjugation of the toxin small molecule to the four cysteines. The selection of mutation sites is the key point in the present invention, and the quality of the site directly determines the properties of the ADC. After the small molecule drug is connected to a site with high solvent accessibility, the possibility of thiol exchange between the small molecule and the protein in the plasma is increased due to the relatively exposed environment of the small molecule drug, thus weakening the efficacy and toxic and side effects. Furthermore, the more exposed small molecules also significantly increase the hydrophobicity of the antibody, thus affecting PK behavior in vivo of the antibody. Thus, the selection of more cryptic sites for engineering may result in more stable and more effective ADC molecules.

It is generally believed that modification in the variable region of an antibody may be associated with a high risk of loss of antigen binding, so in this study we sought sites in the constant region of the antibody. Firstly, the crystal structure of the protein was resolved, and some sites inside the Fab cavity formed by the proteins CH1 and VH, CL and VL, and the cavity formed by the two CH2 and two CH3 domains in the Fc region were selected. The ADC in which small hydrophobic molecules were conjugated at sites within the cavity, thus isolating the small molecules from the hydrophilic environment surrounding the antibody, will thus be more hydrophilic as compared to ADCs in which small molecules were attached to the native interchain disulfide bond.

The candidate mutation sites are shown in the table below, wherein LC205 was a site validated by Genentech, LC110 was disclosed in CN101065151A, HC239 was disclosed in CN110352074A, HC118 was disclosed in CN101065151A, HC375 was disclosed in CN101065151B, and HC442 was disclosed in CN102802661B, which were used as control sites in this study.

| Mutation site | Sequence before mutation | SEQ ID NO |
|---|---|---|
| LLC160 (Eu) | VKAGVETTTPS | 29 |
| LLC166 (Eu) | TTTPSKQSNNK | 30 |
| LC165 (Eu) | QESVTEQDSKD | 31 |
| HC152 (Eu) | KDYFPEPVTVS | 32 |
| HC155 (Eu) | KDYFPEPVTVS | 33 |
| LC138 (Eu) | CLLNNFYPREA | 34 |
| HC290 (Eu) | NAKTKPREEQY | 35 |
| LC167 (Eu) | ESVTEQDSKDS | 36 |
| LC205 (Eu) | GLSSPVTKSFNR | 37 |
| LC160 (Eu) | QSGNSQESVTE | 53 |
| LC166 (Eu) | ESVTEQDSKDS | 54 |
| LC110 (Eu) | RTVAAPSVFIFP | 55 |
| HC239 (Eu) | LLGGPSVFLFP | 56 |
| HC118 (Eu) | ASTKGPSVFPL | 57 |
| HC442 (Eu) | TQKSLSLSPGK | 58 |
| HC375(Eu) | KGFYPSDIAVE | 59 |

### Example 2: Expression and purification of engineered antibody

Expi293F cells (available from Thermo Fisher Scientific) were subcultured in Expi293F cell medium (available from Thermo Fisher Scientific). Cell density was measured the day before transfection and adjusted to 3×10⁶ cells/mL with fresh Expi293F cell medium, followed by the culture, and the cell density was readjusted to 3×10⁶ cells/mL on the day of transfection.

Opti-MEM medium (available from Gibco) with 1/10 of a final volume of transfected Expi293F cell was used as a transfection buffer, 10 µg of the corresponding recombinant plasmid (containing Pcdna3.1 plasmids encoding antibody heavy chain and light chain, respectively, in a ratio of light chain plasmid to heavy chain plasmid of 1:1) was added to each mL of transfection buffer, followed by blending, 30 µg of Polyethyleneimine (PEI) (Polysciences, Cat.: 23966) was added to each mL of transfection buffer, followed by blending, the mixture was incubated at room temperature for 20 min, then the PEI/DNA mixture was poured gently into Expi293F cell suspension, followed by blending, and culturing in a shaker in 8%CO₂, at 36.5°C and 120 rpm.

After culturing for 16-18 h, the culture flask was supplemented with FEED (100 g/L Phyton peptide + 100g/L Difco Select Phyton) at a concentration of 200 g/L in a volume of 1/50 the transfected culture, glucose solution at a final concentration of 5 g/L and VPA (Gibco, Cat.: 11140-050) at a final concentration of 2.2 mM, followed by blending gently, and the mixture was cultured in a shaker in 8%CO₂, at 36.5°C and 120 rpm. After culturing for 6 consecutive days, cultures were collected and centrifuged at 4000 rpm for 30 min, the cell supernatant was filtered through 0.45 µM filter membranes and purified by affinity chromatography and gel chromatography.

The specific operation steps of affinity chromatography purification are as follows: a MabSelect SuRe LX (Cytiva company, Cat.: 17547401) affinity chromatography column was selected, endotoxin was removed from the affinity chromatography column and the tube line with 0.1M NaOH for 2 h before purification, and then the tube line and the column were washed with distilled water. The packed column was equilibrated with 5× column volume of 1× PBS (Gibco, Cat. 10010023); the collected supernatant of the stock solution was passed through the column, and then the packed column was washed with 10× column volume of 1× PBS to remove non-specific binding proteins; the packed material was rinsed with 5× column volume of elution buffer (100 mM citrate, pH 3.3), the eluate was collected, and then the protein pH was adjusted to 6.0 with 2M Tris for further gel chromatography.

The specific gel exchange chromatography purification operation steps are as follows: Superdex 200 Increatase 10/300 GL (GE Healthcare, Cat. 28-9909-44) gel chromatography column was selected and placed in AKTA pure system. Endotoxin was removed from the AKTApure system equipped with Superdex 200 Increatase 10/300 GL gel chromatography column with 0.1M NaOH for 2 h, and then the system and column were washed with distilled water; the column was equilibrated with 2-5 column volumes of 1×PBS until conductivity and pH is stable; and the protein obtained by affinity chromatography was loaded, continuously eluted by using 1×PBS, impurities such as polymers were removed according to ultraviolet absorption peaks, and samples with high purity was collected. The samples were filtered with 0.22 µm membranes, and the protein concentration was determined and detected by SEC.

SDS-PAGE electrophoretic analysis: To a protein sample was added a reducing loading buffer (Yeasen Biotechnology, Cat. 20315ES05) or a non-reducing loading buffer (Beijing BaiaoLaibo, Cat. WE0289), followed by heating at 70°C for 10 min, a proper amount of the sample was added into a prefabricated Gel 4-20% Mini-PROTEAN^{®} TGX^{™} Precast Gel (Bio-rad, Cat. 4561095), a protein marker (Biorad, Cat. 1610375) was added simultaneously as a control, 150V electrophoresis was carried out for 50 min, dyeing by using a protein dyeing instrument (GenScript, L00760C), and then images were taken by using a Gel imaging instrument (Shanghai Tianneng, Tanon-1600).

### Example 3: Preparation of ADC

**I.** Preparation of single-site DAR2 ADC: the single-site mutated antibody expressed in Example 2 was conjugated with linker-drug mc-GGFG-Dxd to give a total of 11 ADC molecules: LLC160-Dxd, LLC166-Dxd, LC165-Dxd, HC152-Dxd, HC155-Dxd, LC138-Dxd, HC290-Dxd, LC167-Dxd, LC205-Dxd, LC160-Dxd, and LC166-Dxd used as controls. In this context, all numbering were based on the Eu numbering scheme, wherein LLC stands for Lambda light chain and LC for Kappa light chain. LLC160-Dxd represents an ADC molecule obtained by conjugation of mc-GGFG-Dxd to a Lambda light chain engineered with cysteine at position 160; LLC166-Dxd represents an ADC molecule obtained by conjugation of mc-GGFG-Dxd to a Lambda light chain engineered with cysteine at position 166; LC165-Dxd represents an ADC molecule obtained by conjugation of mc-GGFG-Dxd to a Kappa light chain engineered with cysteine at position 165; HC152-Dxd represents an ADC molecule obtained by conjugation of mc-GGFG-Dxd to a heavy chain engineered with cysteine at position 152; HC155-Dxd represents an ADC molecule obtained by conjugation of mc-GGFG-Dxd to a heavy chain engineered with cysteine at position 155; LC138-Dxd represents an ADC molecule obtained by conjugation of mc-GGFG-Dxd to a Kappa light chain engineered with cysteine at position 138; HC290-Dxd represents an ADC molecule obtained by conjugation of mc-GGFG-Dxd to a heavy chain engineered with cysteine at position 290; LC167-Dxd represents an ADC molecule obtained by conjugation of mc-GGFG-Dxd to a Kappa light chain engineered with cysteine at position 167; LC205-Dxd represents an ADC molecule obtained by conjugation of mc-GGFG-Dxd to a Kappa light chain engineered with cysteine at position 205; LC160-Dxd represents an ADC molecule obtained by conjugation of mc-GGFG-Dxd to a Kappa light chain engineered with cysteine at position 160; and LC166-Dxd represents an ADC molecule obtained by conjugation of mc-GGFG-Dxd to a Kappa light chain engineered with cysteine at position 166 with mc-GGFG-Dxd.

The antibody was firstly reduced to release the thiol group on the cysteine. During expression of the engineered antibody, cysteine was reacted with cysteine or glutathione in the cell culture medium, thus being sequestered from reacting with the small molecule drug. It is therefore necessary to reduce the antibody to release the thiol group of the cysteine. The antibody was exchanged in a 20 mM histidine solution (Buffer 1) at pH6.5 (PB solution (Buffer 2) at pH7.0 as the conjuation solution for the LC165 site), and 20 mM EDTA and 20-fold molar excess of reducing agent TCEP were added to release free cysteine. The reaction was carried out in a water bath at 37°C for 3 h. After the reaction, the reducing agent and the reduced cysteine or glutathione were removed by overnight dialysis by using a dialysis cup.

Then the interchain disulfide bonds of the antibody were remodeled. In this step, the oxidizing agent dehydroascorbic acid (dhAA) was added at a 40-fold molar ratio and oxidized in a water bath at 37°C for 3 h. The solution was cooled to room temperature, mc-GGFG-Dxd was added at a 12-fold molar ratio, and DMSO was supplemented so that the solution has a volume ratio of DMSO of 10%, and the reaction was carried out at room temperature for 1 h. After adding N-acetylcysteine at a 20-fold molar ratio to the mixture to quench the unreacted small molecule drug for 15 min, the unreacted small molecule drug and other impurities were removed by a Zeba^{™} desalting spin column, and the ADC was exchanged in a 20 mM histidine solution pH5.5 for storage.

**II.** Preparation of dual-site DAR4 ADC: single sites were combined, according to the method of Example 2, an antibody expressing 13 dual-site mutations was used for conjugation with linker-drug mc-GGFG-Dxd, thus resulting in a total of 13 ADC molecules: LLC160-LLC166-Dxd, LLC166-HC152-Dxd, LLC160-HC155-Dxd, LLC166-HC155-Dxd, LC165-HC152-Dxd, LC165-HC155-Dxd, LC160-LC166-Dxd, LC110-HC239-Dxd, LC110-HC118-Dxd, LC110-HC442-Dxd, HC118-HC239-Dxd, HC118-HC442-Dxd, HC239-HC442-Dxd, LC160-178-LC166-Dxd, LC166-HC239-Dxd, LC166-HC375-Dxd, HC239-HC375-Dxd.

In this context, all numberings were based on the Eu numbering scheme. LLC160-LLC166-Dxd represents an ADC molecules obtained by conjugation of mc-GGFG-Dxd to a Lambda light chain engineered with cysteine at positions 160 and 166; LLC166-HC152-Dxd represents an ADC molecules obtained by conjugation of mc-GGFG-Dxd to a Lambda light chain engineered with cysteine at position 166 and a heavy chain engineered with cysteine at position 152; LLC160-HC155-Dxd represents an ADC molecules obtained by conjugation of mc-GGFG-Dxd to a Lambda light chain engineered with cysteine at position 160 and a heavy chain engineered with cysteine at position 155; LLC166-HC155-Dxd represents an ADC molecules obtained by conjugation of mc-GGFG-Dxd to a Lambda light chain engineered with cysteine at position 166 and a heavy chain engineered with cysteine at position 155; LC165-HC152-Dxd represents an ADC molecules obtained by conjugation of mc-GGFG-Dxd to a Kappa light chain engineered with cysteine at position 165 and a heavy chain engineered with cysteine at position 152; LC165-HC155-Dxd represents an ADC molecule obtained by conjugation of mc-GGFG-Dxd to a Kappa light chain engineered with cysteine at position 165 and a heavy chain engineered with cysteine at position 155; LC160-LC166-Dxd represents an ADC molecule obtained by conjugation of mc-GGFG-Dxd to a Kappa light chain engineered with cysteine at positions 160 and 166; LC110-HC239-Dxd represents an ADC molecule obtained by conjugation of mc-GGFG-Dxd to a Kappa light chain engineered with cysteine at position 110 and a heavy chain engineered with cysteine at position 239; LC110-HC118-Dxd represents an ADC molecule obtained by conjugation of mc-GGFG-Dxd to a Kappa light chain engineered with cysteine at position 110 and a heavy chain engineered with cysteine at position 118; LC110-HC442-Dxd represents an ADC molecule obtained by conjugation of mc-GGFG-Dxd to a Kappa light chain engineered with cysteine at position 110 and a heavy chain engineered with cysteine at position 442; HC118-HC239-Dxd represents an ADC molecule obtained by conjugation of mc-GGFG-Dxd to an antibody with a heavy chain engineered with cysteine at positions 118 and 239 to mc-GGFG-Dx, HC118-HC442-Dxd represents an ADC molecule obtained by conjugation of mc-GGFG-Dxd to a heavy chain engineered with cysteine at positions 118 and 442; HC239-HC442-Dxd represents an ADC molecule obtained by conjugation of mc-GGFG-Dxd to a heavy chain engineered with cysteine at positions 239 and 442; LC160-178-LC166-Dxd represents an ADC molecule obtained by conjugation of mc-GGFG-Dxd to a Kappa light chain engineered with cysteine at positions 160 and 166 and having an amino acid mutation from T to Y at position 178; LC166-HC239-Dxd represents an ADC molecule obtained by conjugation of mc-GGFG-Dxd to a Kappa light chain engineered with cysteine at position 166 and a heavy chain engineered with cysteine at position 239; LC166-HC375-Dxd represents an ADC molecule obtained by conjugation of mc-GGFG-Dxd to a Kappa light chain engineered with cysteine at position 166 and a heavy chain engineered with cysteine at position 375; and HC239-HC375-Dxd represents an ADC molecule obtained by conjugation of mc-GGFG-Dxd to a heavy chain engineered with cysteine at positions 239 and 375 with mc-GGFG-Dxd.

In the LC160-178-LC166 antibody, not only mutations to cysteine occur at sites 160 and 166, but also a mutation of T at site 178 in the vicinity of 160 to Y at the site in the vicinity of site LLC160 occurs, thereby changing the amino acid environment in the vicinity of the site LC160 to make the properties of the site LC160 closer to that of LLC160.

### Preparation of LLC160-LLC166-Dxd:

The antibody was firstly reduced to release the thiol group on the cysteine. During the expression of the engineered antibody, cysteine was reacted with cysteine or glutathione in the cell culture medium, thus being blocked and thus cannot react reacting with the small molecule drug. It is therefore necessary to reduce the antibody to release the thiol group of the cysteine. Antibody LLC160-LLC166 was exchanged in a 20 mM histidine solution (Buffer 1) at pH6.5, and 20 mM EDTA and 20-fold molar excess of reducing agent TCEP were added to release free cysteine. The reaction was carried out in a water bath at 37°C for 3 h. After the reaction, the reducing agent and the reduced cysteine or glutathione were removed by overnight dialysis by using a dialysis cup.

Then the interchain disulfide bonds of the antibody were remodeled. In this step, the oxidizing agent dehydroascorbic acid (dhAA) was added at a 40-fold molar ratio and oxidized in a water bath at 37°C for 3 h. The solution was cooled to room temperature, mc-GGFG-Dxd was added at a 12-fold molar ratio, and DMSO was supplemented so that the solution has a volume ratio of DMSO of 10%, and the reaction was carried out at room temperature for 1 h. After adding N-acetylcysteine at a 20-fold molar ratio to the mixture to quench the unreacted small molecule drug for 15 min, the unreacted small molecule drug and other impurities were removed by a Zeba^{™} desalting spin column, and the ADC was exchanged in a 20 mM histidine solution pH5.5 for storage.

LC160-LC166-Dxd, LC110-HC239-Dxd, LC110-HC118-Dxd, LC110-HC442-Dxd, HC118-HC239-Dxd, HC118-HC442-Dxd, HC239-HC442-Dxd, LC160-178-LC166-Dxd, LC166-HC239-Dxd, LC166-HC375-Dxd, and HC239-HC375-Dxd were prepared in a similar way.

### Preparation of LLC166-HC152-Dxd:

Antibody LLC166-HC152 was exchanged in 20mM histidine solution (Buffer 2) at pH7.0, and 20 mM EDTA and 20-fold molar excess of reducing agent TCEP were added to release free cysteine. The reaction was carried out in a water bath at 37°C for 3 h. After the reaction, the reducing agent and the reduced cysteine or glutathione were removed by overnight dialysis by using a dialysis cup.

The oxidizing agent dhAA was added at a 40-fold molar ratio and oxidized in a water bath at 37°C for 3 h for remodeling the interchain disulfide bonds of antibodies. The solution was cooled to room temperature, mc-GGFG-Dxd was added at a 12-fold molar ratio, and DMSO was supplemented so that the solution has a volume ratio of DMSO of 10%, and the reaction was carried out at room temperature for 1 h. After adding N-acetylcysteine at a 20-fold molar ratio to the mixture to quench the unreacted small molecule drug for 15 min, the unreacted small molecule drug and other impurities were removed by a Zeba^{™} desalting spin column, and the ADC was exchanged in a 20 mM histidine solution pH5.5 for storage.

### Preparation of LLC160-HC155-Dxd:

Antibody LLC160-HC155 was exchanged in a 20 mM histidine solution (Buffer 2) at pH7.0, and 20 mM EDTA and 20-fold molar excess of reducing agent TCEP were added to release free cysteine. The reaction was carried out in a water bath at 37°C for 3 h. After the reaction, the reducing agent and the reduced cysteine or glutathione were removed by overnight dialysis by using a dialysis cup. The oxidizing agent dhAA was added at a 40-fold molar ratio and oxidized in a water bath at 37°C for 3 h. The solution was cooled to room temperature, mc-GGFG-Dxd was added at a 12-fold molar ratio, and DMSO was supplemented so that the solution has a volume ratio of DMSO of 10%, and the reaction was carried out at room temperature for 1 h. After adding N-acetylcysteine at a 20-fold molar ratio to the mixture to quench the unreacted small molecule drug for 15 min, the unreacted small molecule drug and other impurities were removed by a Zeba^{™} desalting spin column, and the ADC was exchanged in a 20 mM histidine solution pH5.5 for storage.

### Preparation of LLC166-HC155-Dxd:

Antibody LLC166-HC155 was exchanged in 20 mM histidine solution (Buffer 2) at pH7.0, and 20 mM EDTA and 20-fold molar excess of reducing agent TCEP were added to release free cysteine. The reaction was carried out in a water bath at 37°C for 3 h. After the reaction, the reducing agent and the reduced cysteine or glutathione were removed by overnight dialysis by using a dialysis cup. The oxidizing agent dhAA was added at a 40-fold molar ratio and oxidized in a water bath at 37°C for 3 h. The solution was cooled to room temperature, mc-GGFG-Dxd was added at a 12-fold molar ratio, and DMSO was supplemented so that the solution has a volume ratio of DMSO of 10%, and the reaction was carried out at room temperature for 1 h. After adding N-acetylcysteine at a 20-fold molar ratio to the mixture to quench the unreacted small molecule drug for 15 min, the unreacted small molecule drug and other impurities were removed by a Zeba^{™} desalting spin column, and the ADC was exchanged in a 20 mM histidine solution pH5.5 for storage.

### Preparation of LC165-HC 152-Dxd:

Antibody LC165-HC152 was exchanged in 20 mM histidine solution (Buffer 2) at pH7.0, and 20 mM EDTA and 20-fold molar excess of reducing agent TCEP were added to release free cysteine. The reaction was carried out in a water bath at 37°C for 3 h. After the reaction, the reducing agent and the reduced cysteine or glutathione were removed by overnight dialysis by using a dialysis cup. The oxidizing agent dhAA was added at a 40-fold molar ratio and oxidized in a water bath at 37°C for 3 h. The solution was cooled to room temperature, mc-GGFG-Dxd was added at a 12-fold molar ratio, and DMSO was supplemented so that the solution has a volume ratio of DMSO of 10%, and the reaction was carried out at room temperature for 1 h. After adding N-acetylcysteine to the mixture at a 20-fold molar ratio to quench the unreacted small molecule drug for 15 min, the unreacted small molecule drug and other impurities were removed by a Zeba^{™} desalting spin column, and the ADC was exchanged in a 20 mM histidine solution pH5.5 for storage.

### Preparation of LC165-HC155-Dxd:

Antibody LC165-HC155 was exchanged in 20 mM histidine solution (Buffer 2) at pH7.0, and 20 mM EDTA and 20-fold molar excess of reducing agent TCEP were added to release free cysteine. The reaction was carried out in a water bath at 37°C for 3 h. After the reaction, the reducing agent and the reduced cysteine or glutathione were removed by overnight dialysis by using a dialysis cup. The oxidizing agent dhAA was added at a 40-fold molar ratio and oxidized in a water bath at 37°C for 3 h. The solution was cooled to room temperature, mc-GGFG-Dxd was added at a 12-fold molar ratio, and DMSO was supplemented so that the solution has a volume ratio of DMSO of 10%, and the reaction was carried out at room temperature for 1 h. After adding N-acetylcysteine to the mixture at a 20-fold molar ratio to quench the unreacted small molecule drug for 15 min, the unreacted small molecule drug and other impurities were removed by a Zeba^{™} desalting spin column, and the ADC was exchanged in a 20 mM histidine solution pH5.5 for storage.

### Preparation of TZB-Dxd-DAR4/TZB-Dxd-DAR2:

Randomly conjugated DAR4 ADCs and DAR2 ADCs were prepared as controls. The proportion of reducing agent TCEP added was first tested. TCEPs at different ratios were respectively added in the Trastuzumab antibody solution, the mixture was reacted for 2 h at 25°C, then mc-GGFG-Dxd was added at a 10-fold molar ratio, DMSO was supplemented so that the final system has a volume ratio of DMSO of 10%, and the mixture was reacted for 1 h at 25°C. After adding N-acetylcysteine at a 20-fold molar ratio to quench the unreacted small molecule drug for 15 min, the unreacted small molecule drug and other impurities were removed by a Zeba^{™} desalting spin column, and the ADC was exchanged in a 20 mM histidine solution pH5.5 for storage. DAR was calculated by RP-HPLC, and after linear regression of TCEP with different ratios, the TCEP ratio required for DAR of 4 or DAR of 2 was calculated. Based on the obtained TCEP ratio, ADC molecules with an average DAR of 4 or 2 were prepared according to the above experimental method.

### Preparation of TZB-Dxd-DAR8

DAR8 ADCs were prepared as controls. Firstly, TCEP was added in the Trastuzumab antibody solution at a 20-fold molar ratio, the mixture was reacted for 2 h at 25°C, then mc-GGFG-Dxd was added at a 12-fold molar ratio, DMSO was supplemented so that the final system has a volume ratio of 10%, and the mixture was reacted for 1 h at 25°C. After adding N-acetylcysteine at a 20-fold molar ratio to quench the unreacted small molecule drug for 15 min, the unreacted small molecule drug and other impurities were removed by a Zeba^{™} desalting spin column, and the ADC was exchanged in a 20 mM histidine solution pH5.5 for storage.

### Preparation of negative control IgG-Dxd-DAR4

IgG-Dxd-DAR4 ADCs were prepared as controls. The proportion of reducing agent TCEP added was first tested. TCEPs at different ratio were respectively added to a solution of IgG-anti Hen Egg Lysozyme (Hel) antibodies (heavy chain: SEQ ID NO: 38; light chain: SEQ ID NO: 39), the mixture was reacted for 2 h at 25°C, mc-GGFG-Dxd was added at a 10-fold molar ratio, and DMSO was supplemented so that the final system has a volume ratio of 10%, and the reaction was continued for 1 h at 25°C. After adding N-acetylcysteine at a 20-fold molar ratio to quench the unreacted small molecule drug for 15 min, the unreacted small molecule drug and other impurities were removed by a Zeba^{™} desalting spin column, and the ADC was exchanged in a 20 mM histidine solution pH5.5 for storage. DAR was calculated by RP-HPLC, and after linear regression of TCEP with different ratios, the TCEP ratio required for DAR of 4 was calculated. Based on the obtained TCEP ratio, ADC molecules with an average DAR of 4 were prepared according to the above experimental method.

### Example 4: Analysis on physicochemical properties of ADC

The number of small molecules conjugated to each engineered antibody was analyzed by RP-HPLC. Firstly, a small amount of ADC solution prepared in Example 3 was taken, the pH value was adjusted to 7.5 by using a Tris buffer solution, and a reducing agent DTT was added to completely reduce ADC in a water bath at 37°C. The analysis was then performed by HPLC and the DAR value was calculated from the peak area of each peak at UV280 nm. The analysis conditions were as follows.

| | |
|---|---|
| Equipment | Waters, ACQuity H class UPLC, SZ-A2-DD-HPLC01 |
| Column | Waters, BioResolve RP mAb Polyphenyl, 450 Å, 2.7 µm, 2.1 mm × 15 cm |
| Column Temp. | 70°C |
| Mobile Phase | A: 0.1% TFA in H₂O, B: 0.1% TFA in ACN. |
| Flow Rate | 0.3 mL/min |
| Injection Amount | 10 µg |
| Detection Wavelength | 280 nm; 360 nm |
| Gradient | Isocratic Gradient |

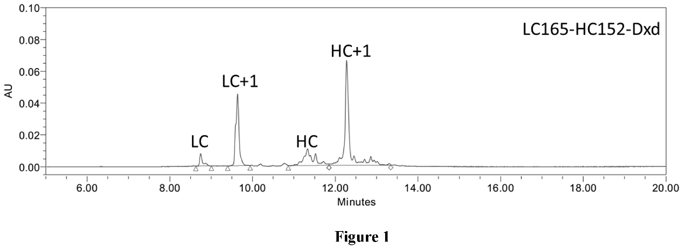

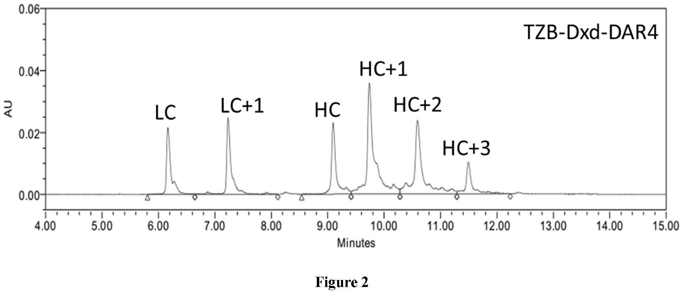

The results are shown in FIGs. 1 and 2.
FIG. 1 shows a representative diagram for the analysis and calculation of site-directed conjugating DAR values. Wherein LC represents the light chain component without the attached drug, LC+1 represents the light chain conjugated with one drug, HC represents the heavy chain component without the attached drug, and HC+1 represents the heavy chain conjugated with one drug.
FIG. 2 shows a representative diagram for the analysis and calculation of random conjugating DAR value. Wherein LC represents the light chain component without the attached drugs, LC+1 represents the light chain conjugated with 1 drug, HC represents the heavy chain component without the attached drugs, HC+1 represents the heavy chain conjugated with one drug, HC+2 represents the heavy chain conjugated with two drugs, and HC+3 represents the heavy chain conjugated with three drugs.

The DAR calculation results for each ADC are shown in Tables 1 and 2 below.

**Table 1: Average DAR of DAR2 ADC:**

| Number | ADC | DAR |
|---|---|---|
| 1 | LLC 160-Dxd | 1.83 |
| 2 | LLC 166-Dxd | 1.85 |
| 3 | LC165-Dxd | 1.50 |
| 4 | HC152-Dxd | 1.50 |
| 5 | HC155-Dxd | 1.54 |
| 6 | LC138-Dxd | 1.87 |
| 7 | HC290-Dxd | 1.67 |
| 8 | LC167-Dxd | 1.50 |
| 9 | LC205-Dxd | 1.81 |
| 10 | LC160-Dxd | 1.72 |
| 11 | LC166-Dxd | 1.80 |

**Table 2: Average DAR of DAR4 ADC:**

| Number | ADC | DAR |
|---|---|---|
| 1 | LLC166-LLC160-Dxd | 3.80 |
| 2 | LLC166-HC152-Dxd | 3.00 |
| 3 | LLC160-HC155-Dxd | 3.51 |
| 4 | LLC166-HC155-Dxd | 3.38 |
| 5 | LC165-HC152-Dxd | 3.25 |
| 6 | LC165-HC155-Dxd | 3.28 |
| 7 | TZB-Dxd-DAR4 | 3.82 |
| 8 | LC110-HC239-Dxd | 3.31 |
| 9 | LC110-HC118-Dxd | 3.32 |
| 10 | LC110-HC442-Dxd | 3.40 |
| 11 | HC118-HC239-Dxd | 3.63 |
| 12 | HC118-HC442-Dxd | 3.55 |
| 13 | HC239-HC442-Dxd | 3.71 |

### ADC Purity Analysis

The purity of the ADC was analyzed using Size Exclusion Chromatography (SEC) according to the conditions in the table below. The purity of ADC was obtained from the ratio of the peak area of the monomer peak, aggregate, and oligomer at UV280 nm.

| Equipment | Agilent, 1260, AS-146 |
|---|---|
| Column | TSK gel G3000SWXL, 7.8 mm × 30 cm, 5 µm |
| Column Temp. | 25°C |
| Mobile Phase | 0.2 M PB, 0.25 M NaCl, 15% (v/v) IPA, pH 7.0 |
| Flow Rate | 0.75 mL/min |
| Injection Amount | 30 µg |
| Detection Wavelength | 280 nm |
| Gradient | Isocratic |
| Time | 18 min |

The purity of each ADC is shown in Tables 3 and 4 below.

**Table 3: Purity of DAR2 ADC:**

| Number | ADC | SEC purity (%) | | |
|---|---|---|---|---|
| | | HMWS | Monomer | LMWS |
| 1 | LLC 160-Dxd | ND | 100 | ND |
| 2 | LLC 166-Dxd | ND | 100 | ND |
| 3 | LC165-Dxd | ND | 100 | ND |
| 4 | HC 152-Dxd | ND | 100 | ND |
| 5 | HC155-Dxd | 0.04 | 99.96 | ND |
| 6 | LC138-Dxd | ND | 100 | ND |
| 7 | HC290-Dxd | ND | 100 | ND |
| 8 | LC167-Dxd | 0.15 | 99.85 | ND |
| 9 | LC205-Dxd | ND | 99.51 | ND |
| 10 | LC160-Dxd | ND | 98.45 | 1.55 |
| 11 | LC166-Dxd | ND | 97.57 | 2.43 |

**Table 4: Purity of DAR4 ADC:**

| Number | ADC | SEC purity (%) | | |
|---|---|---|---|---|
| | | HMWS | Monomer | LMWS |
| 1 | LLC166-LLC160-Dxd | ND | 100 | ND |
| 2 | LLC166-HC152-Dxd | ND | 100 | ND |
| 3 | LLC160-HC155-Dxd | ND | 100 | ND |
| 4 | LLC166-HC155-Dxd | ND | 100 | ND |
| 5 | LC165-HC152-Dxd | 2.84 | 97.16 | ND |
| 6 | LC165-HC155-Dxd | ND | 100 | ND |
| 7 | TZB-Dxd-DAR4 | ND | 100 | ND |
| 8 | LC110-HC239-Dxd | ND | 98.45 | 1.55 |
| 9 | LC110-HC118-Dxd | 0.91 | 99.09 | ND |
| 10 | LC110-HC442-Dxd | 1.77 | 98.28 | ND |
| 11 | HC118-HC239-Dxd | 2.09 | 97.91 | ND |
| 12 | HC118-HC442-Dxd | 1.87 | 98.13 | ND |
| 13 | HC239-HC442-Dxd | 2.00 | 98.00 | ND |

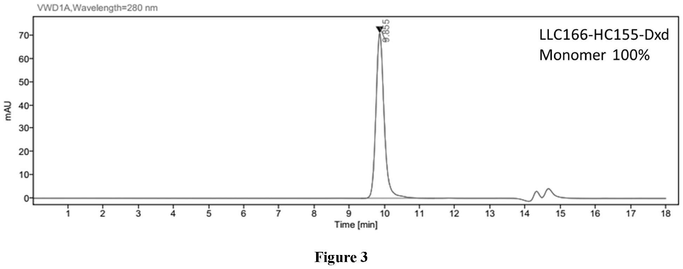

FIG. 3 is a representative diagram for purity analysis (LLC 166-HC 155-Dxd).

As can be seen from the above, all the ADCs have a DAR value of about 3.5 and a purity of more than 95%.

The DAR value and purity of the ADC were determined again according to the analytical method described above. The results are shown in the table below. Each group of ADC has a DAR value of about 3.5, with no significant difference between each group; and a purity of more than 97%, sufficiently for the subsequent comparison of behaviors in vivo and in vitro.

**Table 5: Analysis on physicochemical properties of ADC**

| Number | ADC | DAR | SEC purity (%) | | |
|---|---|---|---|---|---|
| | | | HMWS | Monomer | LMWS |
| 1 | LLC160-LLC166-Dxd | 3.98 | 0.40 | 97.63 | ND |
| 2 | LC160-LC166-Dxd | 3.75 | 0.14 | 99.86 | ND |
| 3 | LC160-178-LC166-Dxd | 3.74 | ND | 99.61 | 0.39 |
| 4 | LC166-HC239-Dxd | 3.74 | 0.20 | 99.80 | ND |
| 5 | LC166-HC375-Dxd | 3.59 | 0.26 | 99.74 | ND |
| 6 | HC239-HC375-Dxd | 3.51 | ND | 100 | ND |

### Analysis on hydrophobicity

The hydrophobicity of ADC was analyzed by Hydrophobic interaction chromatography (HIC) and the analysis conditions were shown in the table below. The hydrophilicity and hydrophobicity of the different ADCs were compared according to the retention time (TR) at UV280 nm.

| Equipment | Agilent, 1260, AS-146 | |
|---|---|---|
| Column | TSKgel Butyl-NPR, 2.5 µm, 4.6 × 100 mm | |
| Column Temp. | 30°C | |
| Mobile Phase | A: 1.5 M ammonium sulfate, 25 mM sodium phosphate, pH 6.0 | |
| | B 25 mM sodium phosphate, pH 6.0 / isopropanol (80:20 v/v) | |
| Flow Rate | 0.65 mL/min | |
| Injection Amount | 30 µg | |
| Detection Wavelength | 280 nm, 360 nm | |
| Time | 41 min | |

| Gradient | Time (min) | Mobile Phase B (%) |
|---|---|---|
| | 0 | 0 |
| | 5 | 0 |
| | 20 | 100 |
| | 25 | 100 |
| | 26 | 0 |
| | 30 | 0 |

The retention times (TR) for the respective ADCs are shown in Tables 6 and 7 below.

**Table 6: Retention Time for DAR2 ADC:**

| Number | ADC | Retention Time (min) |
|---|---|---|
| 1 | LLC 160-Dxd | 12.156 |
| 2 | LLC 166-Dxd | 11.764 |
| 3 | LC165-Dxd | 11.240 |
| 4 | HC152-Dxd | 11.079 |
| 5 | HC155-Dxd | 11.334 |
| 6 | LC205-Dxd | 11.438 |

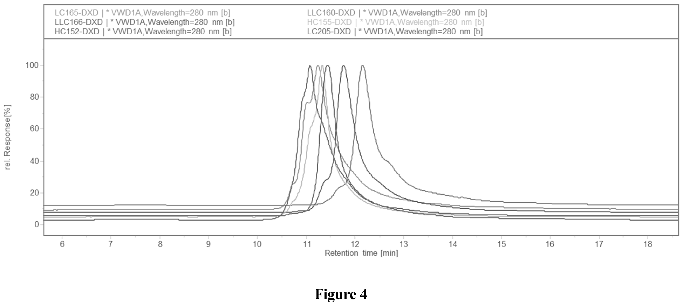

FIG. 4 is a hydrophobic interaction spectrum for each DAR2 ADC.

**Table 7: Retention Time for DAR4 ADC:**

| Number | ADC | Retention Time (min) |
|---|---|---|
| 1 | LLC166-LLC160-Dxd | 11.611 |
| 2 | LLC166-HC152-Dxd | 11.617 |
| 3 | LLC160-HC155-Dxd | 11.854 |
| 4 | LLC166-HC155-Dxd | 11.835 |
| 5 | LC165-HC152-Dxd | 11.221 |
| 6 | LC165-HC155-Dxd | 11.255 |
| 7 | TZB-Dxd-DAR4 | 11.901 |
| 8 | LC110-HC239-Dxd | 12.408 |
| 9 | LC110-HC118-Dxd | 12.346 |
| 10 | LC110-HC442-Dxd | 14.730 |
| 11 | HC118-HC239-Dxd | 12.408 |
| 12 | HC118-HC442-Dxd | 14.809 |
| 13 | HC239-HC442-Dxd | 14.206 |

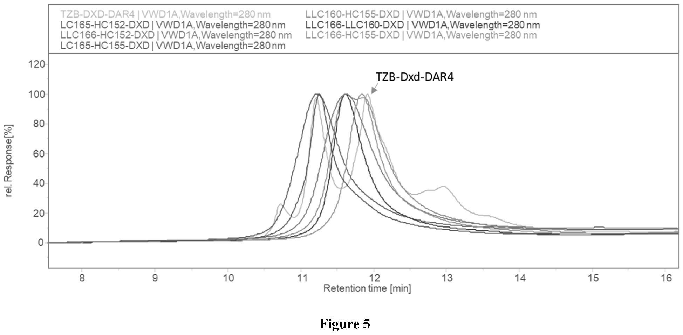

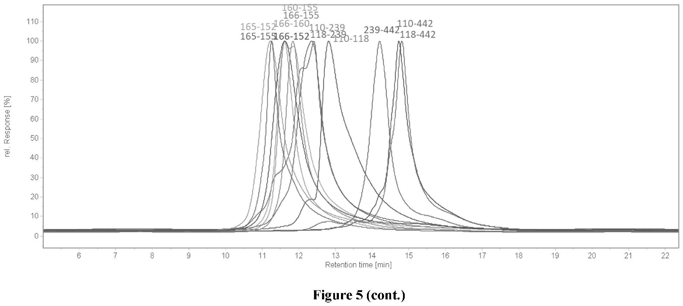

FIG. 5 is a hydrophobic interaction spectrum for each DAR4 ADC showing the retention time of the ADC on the hydrophobic interaction chromatogram.

The intensity of hydrophilicity is generally considered to be a predictor for the PK behavior of ADCs in vivo. ADC molecules with strong hydrophobicity are more likely to aggregate and be removed, thereby reducing the efficacy. The earlier retention time on the HIC spectrum demonstrates a better hydrophilicity. The more posterior peak position demonstrates poor hydrophilicity. As can be seen in FIG. 5 and Table 6, the DAR4 ADCs in groups 1-6 are more hydrophilic than the randomly conjugated TZB-Dxd-DAR4 ADCs. Wherein, LLC160-LLC166-Dxd has a retention time of 11.611 min, and other ADCs of control site combinations have a retention time later than that of LLC160-LLC166-Dxd, wherein HC118-HC442-Dxd has a retention time of 14.809 min, and LC110-HC118-Dxd has an earliest retention time of 12.346 min, which proves that the hydrophilicity of LLC160-LLC166-Dxd has remarkable advantage. It can be predicted that LLC160-LLC166-Dxd with better hydrophilic behavior has excellent pharmacokinetic behavior in vivo, thereby exerting stronger efficacy.

### Example 5: In vitro cell proliferation assay

In vitro cell proliferation assay can be used to evaluate the in vitro activity of ADC molecules. Human mammary adenocarcinoma SK-BR-3 cells (derived from ATCC) were plated in 96-well plates at a density of 2000 per well. The 96-well plates were incubated overnight in a 37°C carbon dioxide incubator.

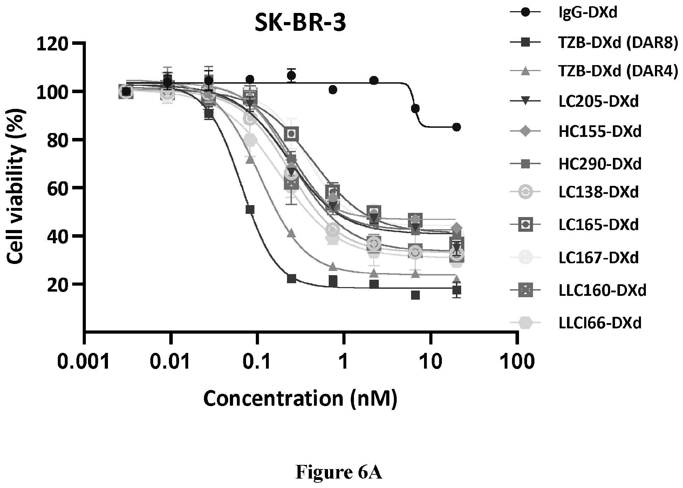

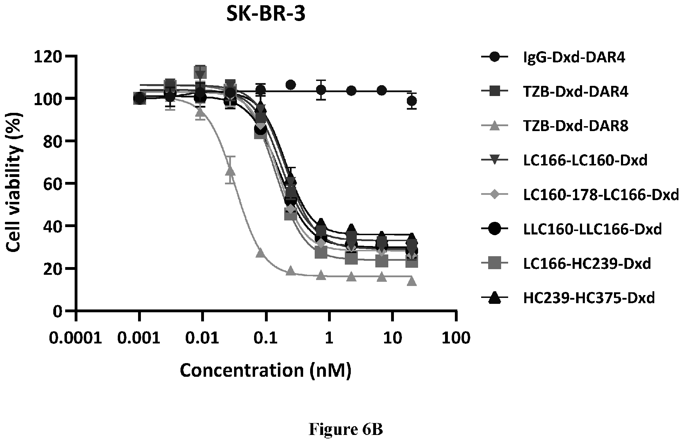

ADC samples were diluted to different concentrations and added sequentially to the well plate, with two replicate wells per sample. The cultivation was continued in the incubator for 6 days. 100 µL CellTiter-Glo^{®} detection reagent was added into each well, followed by uniformly oscillating for 2 min, standing for 10 min at room temperature to stabilize a luminescent signal, the absorbance at 450 nm was detected by using a spectrophotometer, and the results were fitted by using GraphPad Prism software, and EC50 values were calculated. FIGs. 6A and 6B show the cell viability results. Tables 8-1 and 8-2 show the EC50 for killing cells.

**Table 8-1: EC50 for each sample**

| Samples | EC50 (nM) |
|---|---|
| IgG-DXd-DAR4 | NA |
| TZB-DXd (DAR4) | 0.11 |
| TZB-DXd (DAR8) | 0.066 |
| LC205-DXd | 0.30 |
| HC155-DXd | 0.21 |
| HC290-DXd | 0.25 |
| LC138-DXd | 0.24 |
| LC165-DXd | 0.44 |
| LC 167-DXd | 0.38 |
| LLC 160-DXd | 0.27 |
| LLC 166-DXd | 0.18 |

**Table 8-2.**

| Samp les | TZB-Dxd-DAR4 | TZB-Dxd-DAR8 | LC166-LC1 60-Dxd | LC160-178-LC 166-Dxd | LLC160-LLC1 66-Dxd | LC166-HC2 39-Dxd | HC239-HC3 75-Dxd |
|---|---|---|---|---|---|---|---|
| EC50 (nM) | 0.17 | 0.033 | 0.21 | 0.15 | 0.16 | 0.14 | 0.21 |

In the in vitro cell killing experiments, ZB-Dxd-DAR8 has the strongest killing effect, for other groups of ADCs, the killing results are not significantly different, and EC50 values are also relatively close. This is because the DAR values of the ADCs of the respective groups are similar, and the only differences are the differences of the conjugation sites, and the significant difference between the groups cannot be seen due to simple in vitro cell culture environment. However, the difference of different sites will be revealed under the complex physiological environment in vivo.

### Example 6: In vitro binding

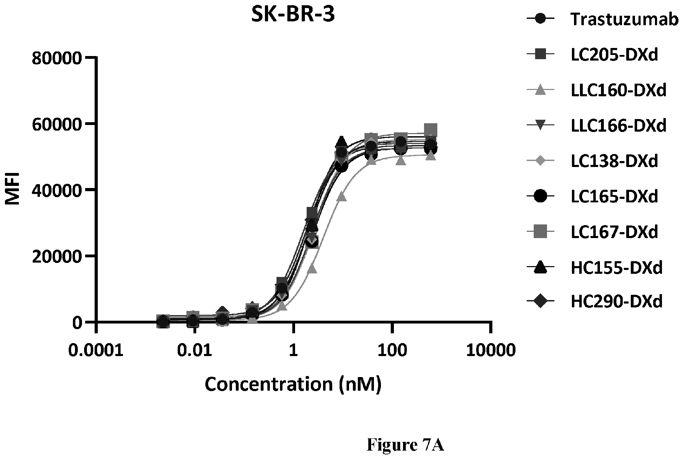

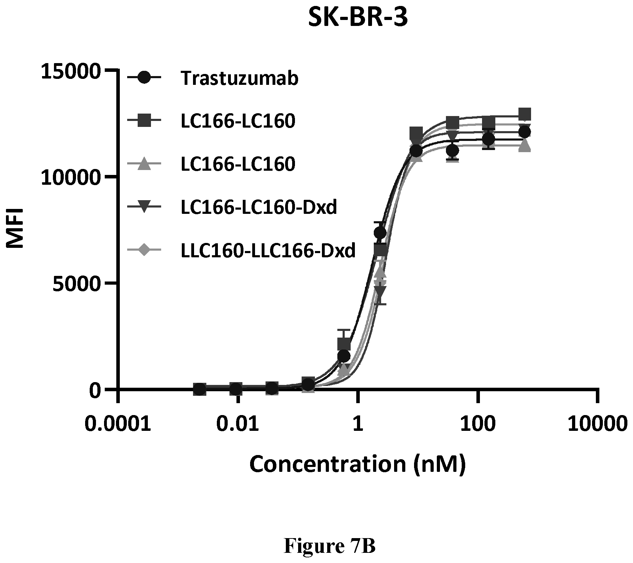

The SK-BR-3 was resuspended in 2 mL FACS buffer, then adjusted to the cell density of 1.5~2.0×10⁶/mL, and added into a 96-well plate at 50 µL/well, and the plate was placed in a refrigerator at 4°C for later use. The test antibody or ADC was diluted in FACS buffer to 200 nM, which was used as the initial concentration for gradient dilution in a 1/3 ratio. The diluted antibody and the cells were mixed in an equal volume of 1:1, and incubated in a refrigerator at 4°C for 30 min; after the incubation, the supernatant was discarded by centrifugation and the cells were washed with FACS buffer. Then, the diluted fluorescent secondary antibody, Goat Anti-Human IgG-PE, was added to the cells, the mixture was resuspended and incubated at 4°C for 30 min in the dark. After centrifugation and washing, the fluorescence value was detected by flow cytometry. Data was processed using FlowJo_V10 and GraphPad Prism 8 software. The results are shown in FIGs. 7A and 7B and Table 9.

**Table 9-1: EC50 of each sample in vitro binding to cells.**

| Samples | EC50 (nM) |
|---|---|
| Trastuzumab | 2.03 |
| LC205-DXd | 1.59 |
| LLC 160-DXd | 4.02 |
| LLC 166-DXd | 2.31 |
| LC138-DXd | 2.58 |
| LC165-DXd | 2.47 |
| LC 167-DXd | 2.83 |
| HC155-DXd | 2.06 |
| HC290-DXd | 1.82 |

**Table 9-2: EC50 of each sample in vitro binding to cells.**

| Sample s | Trastuzuma b | LC166-LC160-Dx d | LC166-LC160-Dx d | LC166-LC160-Dx d | LLC160-LLC166-Dx d |
|---|---|---|---|---|---|
| EC50 (nM) | 1.74 | 2.13 | 2.39 | 2.92 | 2.87 |

As can be seen from FIG. 7A and Table 9-1, each group has similar EC50 values and has little difference compared to Trastuzumab wild-type antibody, indicating that the ability to bind antigen was not affected after conjugation of the mutant antibody to form an ADC.

As can be seen from FIG. 7B and Table 9-2, each group has similar EC50 values and was barely different from Trastuzumab wild-type antibody, indicating that neither antibody mutations nor conjugated ADCs affected the ability to bind antigen. The LLC160-LLC166-Dxd group and LC160-LC160-Dxd group did not differ significantly in terms of cell binding capacity.

### Example 7: Evaluation of stability in plasma

ADC was diluted to 0.2 mg/mL with mouse plasma and the mixture was incubated at 37°C in a carbon dioxide incubator for 0d, 2d, 7d and 14d, and then the ADC was extracted from the plasma by affinity chromatography for further analysis. The extraction was conducted according to the following procedures. Firstly, a gel resin capable of specifically binding to Fc of human IgG (GOAT ANTI-HUMAN IGG -AGAROS Sigma, A3316) was incubated with the sample at room temperature for 30 min. The plasma was removed by centrifugation and unbound proteins were washed away with PBS. Finally, after incubating the resin with citric acid solution of pH3.0 for 10 min, the ADC solution was collected by centrifugation and adjusted to pH 7.5 with Tris. A reducing agent DTT was added to completely reduce ADC in a water bath at 37°C. The DAR values were then analyzed by HPLC and the DAR values for each time point were shown in Tables 9 and 10. Wherein a reduction ratio of DAR over 14 days = (DAR on day 14-initial DAR)/DAR on day 14.

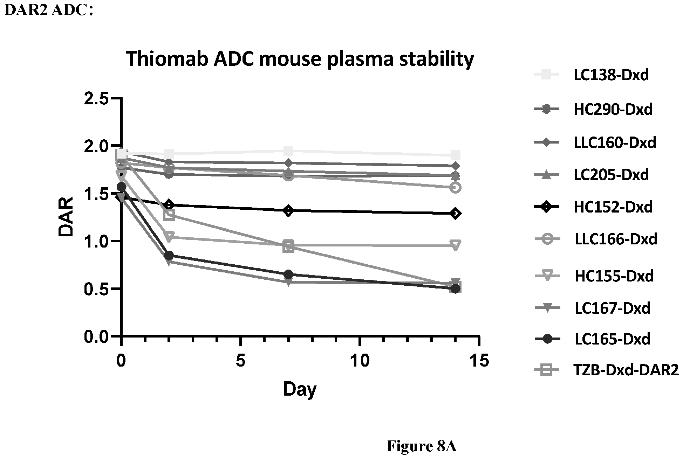

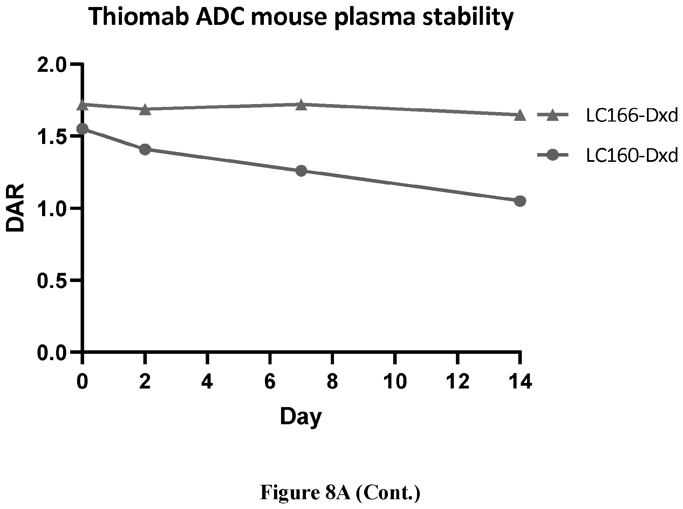

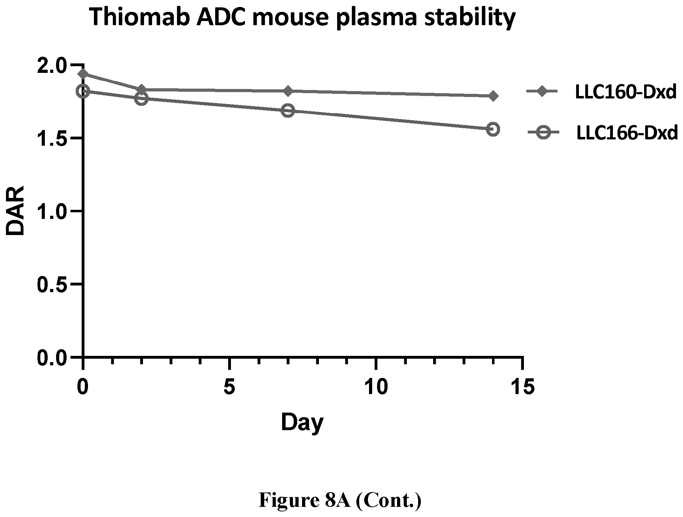

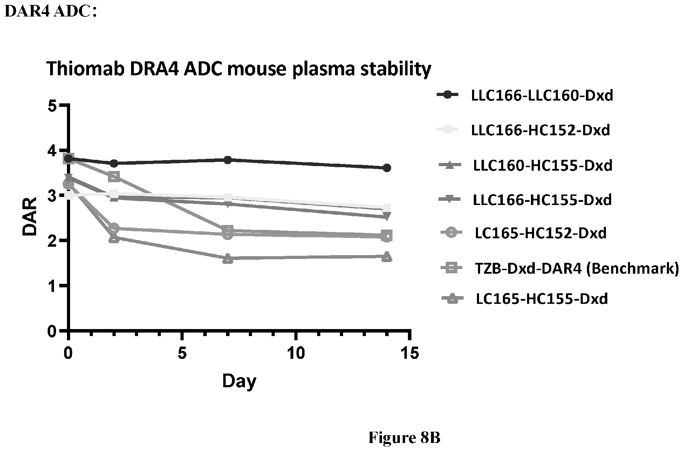

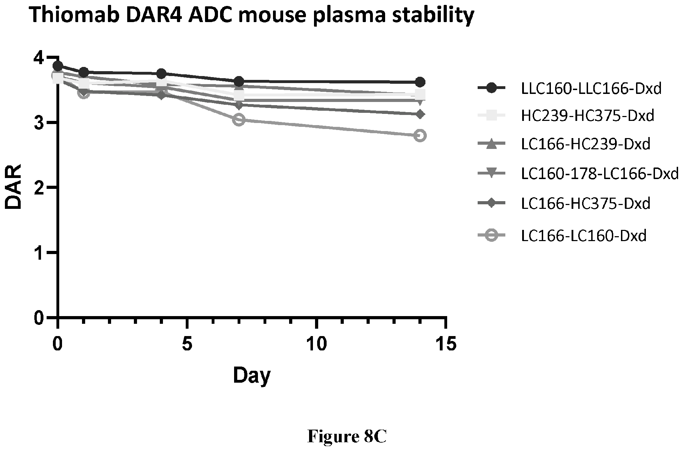

The change profiles of DAR in mouse plasma for each ADC were shown in FIGs. 8A (DAR2 ADC), 8B, and 8C (DAR4 ADC). As can be seen from the DAR2 stability results, all of the site-specificconjugated DAR2 ADCs have better stability than the randomly conjugated DAR2 ADC (TZB-Dxd-DAR2). Among them, LLC160-Dxd is more stable than LC205-Dxd. The DAR value of the LC166-Dxd is only reduced by 4% over 14 days, with a relatively superior stability. LC160-Dxd has an initial DAR of 1.72, which will be reduced to 1.56 at Time point T0 as the drugs rapidly shed once being placed in plasma, and will be reduced to 1.05 after incubation in plasm for 14 days, indicating a relatively poor stability. Based on the results of the comparison of plasma stability of the single-site ADC, it was concluded that the stability of the dual-site combination of LC160-Dxd and LC166-Dxd was overall less stable than that of the dual-site combination of LLC160-Dxd and LLC166-Dxd. That is, LLC160-LLC166-Dxd is more stable in plasma than LC160-LC166-Dxd, thus being more effective.

From the DAR4 stability results (Table 11), it can be seen that TZB-Dxd-DAR4 has a decrease of DAR by 44.5% over 14 days, but LLC166-LLC160-Dxd has a decrease of DAR only by 5.5%, showing certain superiority.

As can be seen from Table 12, LLC160-LLC166-Dxd has a decrease of DAR value only by 6.46% over 14 days, which is the most stable. Whereas the LC160-LC166-Dxd ADC has a rapid decrease of DAR, with a decrease of 24.73% over 14 days. After engineering the LC178 site, the stability of LC160-178-LC166-Dxd is increased significantly, which shows that the amino acid environment around the 160 site is very important for the stability of ADC. It was proved that LLC160-LLC166-Dxd is more stable than LC160-LC166-Dxd in mouse plasma.

**Table 10: Decrease of DAR in mouse plasma for each DAR2 ADC.**

| ADC | Day 0 | Day 2 | Day 7 | Day 14 | 14-day DAR reduction ratio (%) |
|---|---|---|---|---|---|
| LC138-Dxd | 1.92 | 1.91 | 1.95 | 1.90 | 1.04 |
| HC290-Dxd | 1.77 | 1.7 | 1.68 | 1.69 | 4.80 |
| LLC160 -Dxd | 1.94 | 1.83 | 1.82 | 1.79 | 7.78 |
| LC205-Dxd | 1.88 | 1.77 | 1.73 | 1.69 | 9.87 |
| HC152-Dxd | 1.46 | 1.38 | 1.32 | 1.29 | 11.64 |
| LLC 166-Dxd | 1.82 | 1.77 | 1.69 | 1.56 | 14.23 |
| HC155-Dxd | 1.68 | 1.04 | 0.96 | 0.95 | 43.27 |
| LC167-Dxd | 1.46 | 0.79 | 0.57 | 0.56 | 61.54 |
| LC165-Dxd | 1.57 | 0.85 | 0.65 | 0.50 | 68.03 |
| TZB-Dxd-DAR2 | 1.9 | 1.28 | 0.94 | 0.52 | 72.53 |
| LC160-Dxd | 1.56 | 1.41 | 1.26 | 1.05 | 32.26 |
| LC166-Dxd | 1.72 | 1.69 | 1.72 | 1.65 | 4.07 |

**Table 11: Decrease of DAR in mouse plasma for each DAR4 ADC.**

| ADC | Day 0 | Day 2 | Day 7 | Day 14 | DAR reduction ratio |
|---|---|---|---|---|---|
| LLC166-LLC160-Dxd | 3.82 | 3.71 | 3.79 | 3.61 | 5.5 |
| LLC166-HC152-Dxd | 3.00 | 3.04 | 2.96 | 2.73 | 9.0 |
| LLC160-HC155-Dxd | 3.41 | 2.96 | 2.95 | 2.7 | 20.8 |
| LLC166-HC155-Dxd | 3.39 | 2.94 | 2.81 | 2.52 | 25.7 |
| LC165-HC152-Dxd | 3.25 | 2.27 | 2.14 | 2.08 | 36.0 |
| TZB-Dxd-DAR4 (Benchmark DAR4) | 3.82 | 3.42 | 2.22 | 2.12 | 44.5 |
| LC165-HC155-Dxd | 3.28 | 2.07 | 1.61 | 1.65 | 49.7 |

**Table 12: Decrease of DAR in mouse plasma for each DAR4 ADC.**

| Number | ADC | T0 | D1 | D4 | D7 | D14 | DAR reduction ratio (%) |
|---|---|---|---|---|---|---|---|
| 1 | LLC160-LLC166-Dxd | 3.87 | 3.77 | 3.75 | 3.63 | 3.62 | 6.46 |
| 2 | HC239-HC375-Dxd | 3.68 | 3.6 | 3.64 | 3.42 | 3.43 | 6.79 |
| 3 | LC166-HC239-Dxd | 3.77 | 3.70 | 3.58 | 3.56 | 3.42 | 9.28 |
| 4 | LC160-178-LC166-Dxd | 3.7 | 3.61 | 3.55 | 3.34 | 3.34 | 9.73 |
| 5 | LC166-HC375-Dxd | 3.66 | 3.48 | 3.42 | 3.27 | 3.13 | 14.48 |
| 6 | LC166-LC160-Dxd | 3.72 | 3.46 | 3.47 | 3.04 | 2.8 | 24.73 |

### Example 8: In vivo efficacy evaluation

Transgenic mice with high expression of Her2 were used to determine the in vivo efficacy of ADCs. Human gastric cancer cells NCI-N87 (purchased from ATCC) were subcutaneously transplanted into CB17-SCID mice (6-8 weeks old, Beijing Vital River Laboratory Animal Technology Co., Ltd.), which were inoculated with 2×10⁶ cells; the tumor size was measured and monitored using a vernier caliper and average tumor volume was calculated.

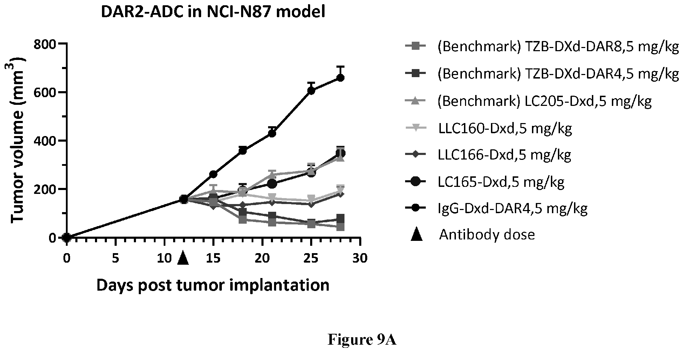

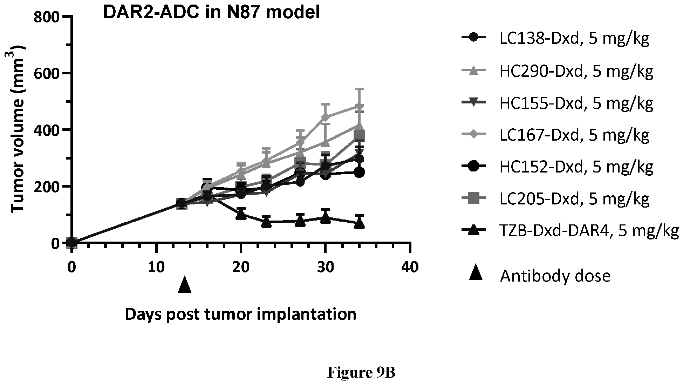

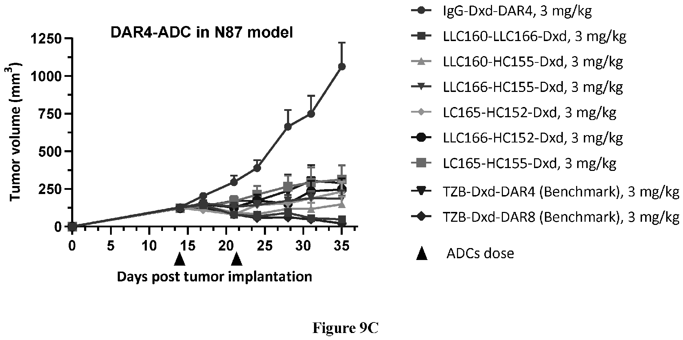

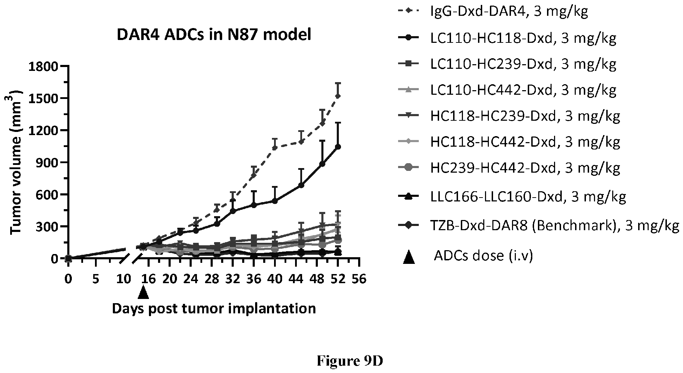

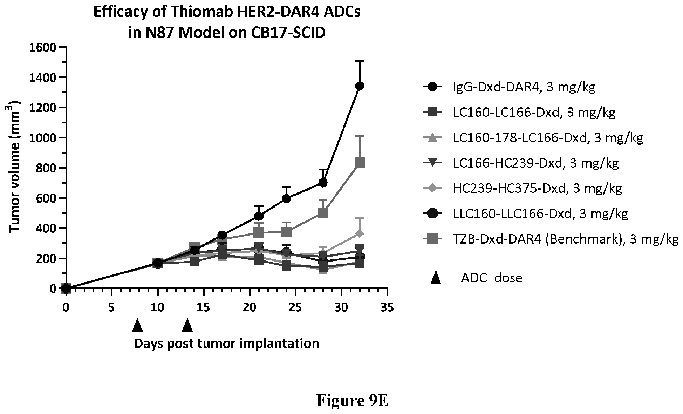

Mice were randomized (n =5) on day 12 and administrated once at the dose shown in FIG. 9 (where FIGs. 9A-9C and 9E show peritoneal administration and FIG. 9D shows intravenous injection). The tumor volume was measured every three days for each administration group. The results are shown in FIG. 9.

The efficacy result of DAR2 ADC showed that TZB-Dxd-DAR8 exhibited the strongest tumor inhibitory effect. TZB-Dxd-DAR4 (traditional conjugating) exhibited the least effective among each experimental group. The LLC160-Dxd group and LLC166-Dxd group showed the best efficacy, which were superior to that of the control group LC205-Dxd. The efficacy results of DAR4 ADC showed that all the site-specifically conjugated DAR4 ADC had an anti-tumor activity between that of TZB-Dxd-DAR8 and TZB-Dxd-DAR4, and each group of ADCs was more effective than TZB-Dxd-DAR4. Amon them, LLC166-LLC160-Dxd and LC160-LC166-Dxd showed similar pharmacodynamic activity (no significant difference), which showed the best efficacy, and superior to that of other controls LC110-HC239-Dxd, LC110-HC118-Dxd, LC110-HC442-Dxd, HC118-HC239-Dxd, HC118-HC442-Dxd, HC239-HC442-Dxd, similarly to efficacy of TZB-Dxd-DAR8 ADC. It was proved the in vivo efficacysuperiority of LLC160-LLC166-Dxd.

### Sequence listing

| SEQ ID NO | Description | Sequence |
|---|---|---|
| 1 | CH1 domain | |
| 2 | Human IgG1 Fc region polypeptide | |
| 3 | Human IgG1 heavy chain constant region | |
| 4 | Human IgG4 Fc region polypeptide | PPCPSCPAPEFLGGPSVFLFPPKPKDTLMISRTPEVTCVVVDVSQEDPEVQ FNWYVDGVEVHNAKTKPREEQFNSTYRVVSVLTVLHQDWLNGKEYKC KVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEG NVFSCSVMHEALHNHYTQKSLSLSLGK |
| 5 | Human Kappa light chain CL region | |
| 6 | Human Lambda light chain CL region | GQPKAAPSVTLFPPSSEELQANKATLVCLISDFYPGAVTVAWKADSSPVK AGVETTTPSKQSNNKYAASSYLSLTPEQWKSHRSYSCQVTHEGSTVEKT VAPTECS |
| 7 | Sequence in the vicinity of LLC160 (Eu) mutation | VKAGVCTTTPS |
| 8 | Sequence in the vicinity of LLC166 (Eu) mutation | TTTPSCQSNNK |
| 9 | Sequence in the vicinity of LC165 (Eu) mutation | QESVTCQDSKD |
| 10 | Sequence in the vicinity of | KDYFPCPVTVS |

| | | |
|---|---|---|
| | HC152 (Eu) mutation | |
| 11 | Sequence in the vicinity of HC155 (Eu) mutation | KDYFPEPVCVS |
| 12 | Sequence in the vicinity of LC138 (Eu) mutation | CLLNCFYPREA |
| 13 | Sequence in the vicinity of HC290 (Eu) mutation | NAKTCPREEQY |
| 14 | Sequence in the vicinity of LC167 (Eu) mutation | ESVTEQCSKDS |
| 15 | Sequence in the vicinity of LC205 (Eu) mutation | GLSSPCTKSFNR |
| 16 | Lambda light chain constant region (LLC160 (Eu)) | GQPKAAPSVTLFPPSSEELQANKATLVCLISDFYPGAVTVAWKADSSPVK AGVCTTTPSKQSNNKYAASSYLSLTPEQWKSHRSYSCQVTHEGSTVEKT VAPTECS |
| 17 | Lambda light chain constant region (LLC166 (Eu)) | GQPKAAPSVTLFPPSSEELQANKATLVCLISDFYPGAVTVAWKADSSPVK AGVETTTPSCQSNNKYAASSYLSLTPEQWKSHRSYSCQVTHEGSTVEKT VAPTECS |
| 18 | Lambda light chain constant region (LLC160-LL C166 (Eu)) | GQPKAAPSVTLFPPSSEELQANKATLVCLISDFYPGAVTVAWKADSSPVK AGVCTTTPSCQSNNKYAASSYLSLTPEQWKSHRSYSCQVTHEGSTVEKT VAPTECS |
| 19 | Kappa light chain constant region (LC138 (Eu)) | RTVAAPSVFIFPPSDEQLKSGTASVVCLLNCFYPREAKVQWKVDNALQS GNSQESVTEQDSKDSTYSLSSTLTLSKADYEKHKVYACEVTHQGLSSPV TKSFNRGEC |
| 20 | Kappa light chain constant region | RTVAAPSVFIFPPSDEQLKSGTASVVCLLNNFYPREAKVQWKVDNALQS GNSQESVTCQDSKDSTYSLSSTLTLSKADYEKHKVYACEVTHQGLSSPV TKSFNRGEC |

| | | |
|---|---|---|
| | (LC165 (Eu)) | |
| 21 | Kappa light chain constant region (LC167 (Eu)) | RTVAAPSVFIFPPSDEQLKSGTASVVCLLNNFYPREAKVQWKVDNALQS GNSQESVTEQCSKDSTYSLSSTLTLSKADYEKHKVYACEVTHQGLSSPV TKSFNRGEC |
| 22 | IgG1 CH1(HC152 (Eu)) | ASTKGPSVFPLAPSSKSTSGGTAALGCLVKDYFPCPVTVSWNSGALTSG VHTFPAVLQSSGLYSLSSVVTVPSSSLGTQTYICNVNHKPSNTKVDKKVE PKSC |
| 23 | IgG1 CH1(HC155 (Eu)) | ASTKGPSVFPLAPSSKSTSGGTAALGCLVKDYFPEPVCVSWNSGALTSG VHTFPAVLQSSGLYSLSSVVTVPSSSLGTQTYICNVNHKPSNTKVDKKVE PKSC |
| 24 | IgG1 Fc(HC290 (Eu)) | DKTHTCPPCPAPELLGGPSVFLFPPKPKDTLMISRTPEVTCVVVDVSHED PEVKFNWYVDGVEVHNAKTCPREEQYNSTYRVVSVLTVLHQDWLNGK EYKCKVSNKALPAPIEKTISKAKGQPREPQVYTLPPSRDELTKNQVSLTC LVKGFYPSDIAVEWESNGQPENNYKTTPPVLDSDGSFFLYSKLTVDKSR WQQGNVFSCSVMHEALHNHYTQKSLSLSPGK |
| 25 | IgG1 constant region (HC152 (Eu)) | ASTKGPSVFPLAPSSKSTSGGTAALGCLVKDYFPCPVTVSWNSGALTSG VHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTV DKSRWQQGNVFSCSVMHEALHNHYTQKSLSLSPGK |
| 26 | IgG1 constant region (HC155 (Eu)) | ASTKGPSVFPLAPSSKSTSGGTAALGCLVKDYFPEPVCVSWNSGALTSG VHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTV DKSRWQQGNVFSCSVMHEALHNHYTQKSLSLSPGK |
| 27 | IgG1 constant region (HC290 (Eu)) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDV SHEDPEVKFNWYVDGVEVHNAKTCPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTV DKSRWQQGNVFSCSVMHEALHNHYTQKSLSLSPGK |
| 28 | Sequence in the vicinity of LLC160-166 (Eu) mutations | VKAGVCTTTPSCQSNNK |
| 38 | IgG-anti hel LC | MDFQVQIISFLLISASVIMSRGDIQMTQSPSSLSASVGDRVTITCRASQSIS SYLNWYQQKPGKAPKLLIYAASSLQSGVPSRFSGSGSGTDFTLTISSLQP |

| | | |
|---|---|---|
| | | EDFATYYCQQADLPAFAFGGGTKVEIKRTVAAPSVFIFPPSDEQLKSGTA SVVCLLNNFYPREAKVQWKVDNALQSGNSQESVTEQDSKDSTYSLSST LTLSKADYEKHKVYACEVTHQGLSSPVTKSFNRGEC |
| 39 | IgG-anti hel HC | MGWSLILLFLVAVATRVLSEVRLLESGGGLVQPGGSLRLSCAASGFTFSN YAMGWVRQAPGKGLEWVSAISGSGGSTYYADSVKGRFTTSRDDSKNA LYLQMNSLRAEDTAVYYCARGGPGWYAADVWGQGTTVTVSSASTKGP SVFPLAPSSKSTSGGTAALGCLVKDYFPEPVTVSWNSGALTSGVHTFPAV LQSSGLYSLSSVVTVPSSSLGTQTYICNVNHKPSNTKVDKKAEPKSCDK THTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKE YKCKVSNKALPAPIEKTISKAKGQPREPQVYTLPPSRDELTKNQVSLTCL VKGFYPSDIAVEWESNGQPENNYKTTPPVLDSDGSFFLYSKLTVDKSRW QQGNVFSCSVMHEALHNHYTQKSLSLSPGK |
| 40 | Sites in the vicinity of LC160(Eu) mutation | QSGNSCESVTE |
| 41 | Sites in the vicinity of LC166(Eu) mutation | ESVTECDSKDS |
| 42 | Sites in the vicinity of LC160-LC166 (Eu) mutations | SGNSCESVTECDSKDS |
| 43 | Kappa light chain constant region (LC160 (Eu)) | RTVAAPSVFIFPPSDEQLKSGTASVVCLLNNFYPREAKVQWKVDNALQS GNSCESVTEQDSKDSTYSLSSTLTLSKADYEKHKVYACEVTHQGLSSPV TKSFNRGEC |
| 44 | Kappa light chain constant region (LC166 (Eu)) | RTVAAPSVFIFPPSDEQLKSGTASVVCLLNNFYPREAKVQWKVDNALQS GNSQESVTECDSKDSTYSLSSTLTLSKADYEKHKVYACEVTHQGLSSPV TKSFNRGEC |
| 45 | Kappa light chain constant region (LC160-LC16 6 (Eu)) | RTVAAPSVFIFPPSDEQLKSGTASVVCLLNNFYPREAKVQWKVDNALQS GNSCESVTECDSKDSTYSLSSTLTLSKADYEKHKVYACEVTHQGLSSPV TKSFNRGEC |
| 46 | Kappa light chain constant region (LC110 (Eu)) | RTCAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC |
| 47 | IgG1 heavy | CSTKGPSVFPLAPSSKSTSGGTAALGCLVKDYFPEPVTVSWNSGALTSG |

| | | |
|---|---|---|
| | chain constant region (HC118 (Eu)) | VHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTV DKSRWQQGNVFSCSVMHEALHNHYTQKSLSLSPGK |
| 48 | IgG1 heavy chain constant region (HC239 (Eu)) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVE PKSCDKTHTCPPCPAPELLGGPCVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTV DKSRWQQGNVFSCSVMHEALHNHYTQKSLSLSPGK |
| 49 | IgG1 heavy chain constant region (HC442 (Eu)) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTV DKSRWQQGNVFSCSVMHEALHNHYTQKSLCLSPGK |
| 50 | IgG1 heavy chain constant region (HC118-HC2 39 (Eu)) | CSTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVE PKSCDKTHTCPPCPAPELLGGPCVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTV DKSRWQQGNVFSCSVMHEALHNHYTQKSLSLSPGK |
| 51 | IgG1 heavy chain constant region (HC118-HC4 42 (Eu)) | CSTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTV DKSRWQQGNVFSCSVMHEALHNHYTQKSLCLSPGK |
| 52 | IgG1 heavy chain constant region (HC239-HC4 42 (Eu)) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVE PKSCDKTHTCPPCPAPELLGGPCVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTV DKSRWQQGNVFSCSVMHEALHNHYTQKSLCLSPGK |
| 60 | IgG1 light chain constant region | DIQMTQSPSSLSASVGDRVTITCRASQDVNTAVAWYQQKPGKAPKLLIY SASFLYSGVPSRFSGSRSGTDFTLTISSLQPEDFATYYCQQHYTTPPTFGQ GTKVEIKRTVAAPSVFIFPPSDEQLKSGTASVVCLLNNFYPREAKVQWK |

| | | |
|---|---|---|
| | (LC160-178-L C166-(Eu)) | VDNALQSGNSCESVTECDSKDSTYSLSSYLTLSKADYEKHKVYACEVT HQGLSSPVTKSFNRGEC |
| 61 | IgG1 heavy chain constant region (HC375-(Eu)) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPCDIAVEWESNGQPENNYKTTPPVLDSDGSFFLYSKLTV DKSRWQQGNVFSCSVMHEALHNHYTQKSLSLSPGK |
| 62 | Sequence in the vicinity of LC160-178-L C166 (Eu) mutation | SCESVTECDSKDSTYSLSSY |
| 63 | IgG1 Fc(HC375 (Eu)) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNG KEYKCKVSNKALPAPIEKTISKAKGQPREPQVYTLPPSRDELTKNQVSLT CLVKGFYPCDIAVEWESNGQPENNYKTTPPVLDSDGSFFLYSKLTVDKS RWQQGNVFSCSVMHEALHNHYTQKSLSLSPGK |
| 64 | IgG1 heavy chain constant region (HC239-HC3 75(Eu)) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVE PKSCDKTHTCPPCPAPELLGGPCVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPCDIAVEWESNGQPENNYKTTPPVLDSDGSFFLYSKLTV DKSRWQQGNVFSCSVMHEALHNHYTQKSLSLSPGK |
| 65 | IgG1 Fc(HC239-H C375 (Eu)) | DKTHTCPPCPAPELLGGPCVFLFPPKPKDTLMISRTPEVTCVVVDVSHED PEVKFNWYVDGVEVHNAKTKPREEQYNSTYRVVSVLTVLHQDWLNG KEYKCKVSNKALPAPIEKTISKAKGQPREPQVYTLPPSRDELTKNQVSLT CLVKGFYPCDIAVEWESNGQPENNYKTTPPVLDSDGSFFLYSKLTVDKS RWQQGNVFSCSVMHEALHNHYTQKSLSLSPGK |
| 66 | Sequence in the vicinity of LC166 (Eu) mutation | ESVTECDSKDS |
| 67 | Sequence in the vicinity of HC375 (Eu) mutation | KGFYPCDIAVE |
| 68 | Sequence in the vicinity of HC239 (Eu) mutation | LLGGPCVFLFP |

## Claims

1. A cysteine-engineered antibody or antigen-binding fragment thereof, wherein
(i) the antibody or antigen-binding fragment thereof comprises a modified Lambda light chain constant region, wherein the amino acid at position 160 of the modified Lambda light chain constant region is mutated to a cysteine as compared to a parent Lambda constant region;
(ii) the antibody or antigen-binding fragment thereof comprises a modified Lambda light chain constant region, wherein the amino acids at positions 160 and 166 of the modified Lambda light chain constant region are mutated to cysteines as compared to a parent Lambda constant region;
(iii) the antibody or antigen-binding fragment thereof comprises a modified Lambda light chain constant region and a modified heavy chain constant region, wherein the amino acid at position 160 of the modified Lambda light chain constant region is mutated to a cysteine as compared to a parent Lambda constant region, and the amino acid at position 152 or 155 of the modified heavy chain constant region is mutated to a cysteine as compared to a parent heavy chain constant region;
(iv) the antibody or antigen-binding fragment thereof comprises a modified Lambda light chain constant region and a modified heavy chain constant region, wherein the amino acid at position 166 of the modified Lambda light chain constant region is mutated to cysteine as compared to a parent Lambda constant region, and the amino acid at position 152 or 155 of the modified heavy chain constant region is mutated to cysteine as compared to a parent heavy chain constant region;
(v) the antibody or antigen-binding fragment thereof comprises a modified Kappa light chain constant region and a modified heavy chain constant region, wherein the amino acid at position 165 of the modified Kappa light chain constant region is mutated to cysteine as compared to a parent Kappa light chain constant region, and the amino acid at position 152 or 155 of the modified heavy chain constant region is mutated to cysteine as compared to a parent heavy chain constant region;
(vi) the antibody or antigen-binding fragment thereof comprises a modified Kappa light chain constant region, wherein the amino acids at positions 160 and 166 of the modified Kappa light chain constant region are mutated to cysteines, and the amino acid at position 178 is mutated to tyrosine, as compared to a parental Kappa constant region;
(vii) the antibody or antigen-binding fragment thereof comprises a modified Kappa light chain constant region and a modified heavy chain constant region, wherein the amino acid at position 166 of the modified Kappa light chain constant region is mutated to cysteine as compared to a parent Kappa constant region, and the amino acid at position 375 of the modified heavy chain constant region is mutated to cysteine as compared to a parent heavy chain constant region; or
(viii) the antibody or antigen-binding fragment thereof comprises a modified heavy chain constant region, wherein the amino acids at positions 239 and 375 of the modified heavy chain constant region are mutated to cysteines as compared to a parent heavy chain constant region.

2. The antibody or antigen-binding fragment thereof according to claim 1, wherein the parent heavy chain constant region is a human IgG1 heavy chain constant region, e.g., comprising the amino acid sequence set forth in SEQ ID NO: 3, or an amino acid sequence having at least 90%, 91%, 92%, 93%, 94%, 95%, 96%, 97%, 98%, or 99% sequence identity to the amino acid sequence set forth in SEQ ID NO: 3 and comprising no mutation to cysteine.

3. The antibody or antigen-binding fragment thereof according to claim 1 or 2, wherein the parent Lambda light chain constant region is a human Lambda light chain constant region, e.g., comprising the amino acid sequence set forth in SEQ ID NO: 6, or an amino acid sequence having at least 90%, 91%, 92%, 93%, 94%, 95%, 96%, 97%, 98%, or 99% sequence identity to the amino acid sequence set forth in SEQ ID NO: 6 and comprising no mutation to cysteine; or
wherein the parent Kappa light chain constant region is a human Kappa light chain constant region, e.g., comprising the amino acid sequence set forth in SEQ ID NO: 5, or an amino acid sequence having at least 90%, 91%, 92%, 93%, 94%, 95%, 96%, 97%, 98%, or 99% sequence identity to the amino acid sequence set forth in SEQ ID NO: 5 and comprising no mutation to cysteine.

4. The antibody or antigen-binding fragment thereof according to any one of claims 1-3, wherein
in item (i) the modified Lambda light chain constant region comprises the amino acid sequence set forth in SEQ ID NO: 7;
in item (ii) the modified Lambda light chain constant region comprises the amino acid sequence set forth in SEQ ID NO: 28;
in item (iii) the modified Lambda light chain constant region comprises the amino acid sequence set forth in SEQ ID NO: 7, and the modified heavy chain constant region comprises the amino acid sequence set forth in SEQ ID NO: 10 or 11;
in item (iv) the modified Lambda light chain constant region comprises the amino acid sequence set forth in SEQ ID NO: 8 and the modified heavy chain constant region comprises the amino acid sequence set forth in SEQ ID NO: 10 or 11;
in item (v) the modified Kappa light chain constant region comprises the amino acid sequence set forth in SEQ ID NO: 9 and the modified heavy chain constant region comprises the amino acid sequence set forth in SEQ ID NO: 10 or 11;
in item (vi) the modified Kappa light chain constant region comprises the amino acid sequence set forth in SEQ ID NO: 62;
in item (vii) the modified Kappa light chain constant region comprises the amino acid sequence set forth in SEQ ID NO: 66 and the modified heavy chain constant region comprises the amino acid sequence set forth in SEQ ID NO: 67; or
in item (viii) the modified heavy chain constant region comprises the amino acid sequence set forth in SEQ ID NO: 67 and the amino acid sequence set forth in SEQ ID NO: 68.

5. The antibody or antigen-binding fragment thereof according to any one of claims 1-4, wherein
in item (i) the modified light chain constant region comprises or consists of the following amino acid sequences: the amino acid sequence set forth in SEQ ID NO: 16, or an amino acid sequence having at least 90%, 91%, 92%, 93%, 94%, 95%, 96%, 97%, 98%, or 99% sequence identity to the amino acid sequence set forth in SEQ ID NO: 16 and having a substitution to cysteine at position 160, or an amino acid sequence having at least 90%, 91%, 92%, 93%, 94%, 95%, 96%, 97%, 98%, or 99% sequence identity to the amino acid sequence set forth in SEQ ID NO: 16 and comprising the amino acid sequence set forth in SEQ ID NO: 7; or
in item (ii) the modified light chain constant region comprises or consists of the following amino acid sequences: the amino acid sequence set forth in SEQ ID NO: 18, or an amino acid sequence having at least 90%, 91%, 92%, 93%, 94%, 95%, 96%, 97%, 98%, or 99% sequence identity to the amino acid sequence set forth in SEQ ID NO: 18 and having substitutions to cysteine at positions 160 and 166, or an amino acid sequence having at least 90%, 91%, 92%, 93%, 94%, 95%, 96%, 97%, 98%, or 99% sequence identity to the amino acid sequence set forth in SEQ ID NO: 18 and comprising the amino acid sequence set forth in SEQ ID NO: 28; or
in item (iii) the modified light chain constant region comprises or consists of the following amino acid sequences: the amino acid sequence set forth in SEQ ID NO: 16, or an amino acid sequence having at least 90%, 91%, 92%, 93%, 94%, 95%, 96%, 97%, 98%, or 99% sequence identity to the amino acid sequence set forth in SEQ ID NO: 16 and having a substitution to cysteine at position 160, or an amino acid sequence having at least 90%, 91%, 92%, 93%, 94%, 95%, 96%, 97%, 98%, or 99% sequence identity to the amino acid sequence set forth in SEQ ID NO: 16 and comprising the amino acid sequence set forth in SEQ ID NO: 7; and
the modified heavy chain constant region comprises or consists of the following amino acid sequences:
the amino acid sequence set forth in SEQ ID NO: 25, or an amino acid sequence having at least 90%, 91%, 92%, 93%, 94%, 95%, 96%, 97%, 98%, or 99% sequence identity to the amino acid sequence set forth in SEQ ID NO: 25 and having a substitution to cysteine at position 152, or an amino acid sequence having at least 90%, 91%, 92%, 93%, 94%, 95%, 96%, 97%, 98%, or 99% sequence identity to the amino acid sequence set forth in SEQ ID NO: 25 and comprising the amino acid sequence set forth in SEQ ID NO: 10; or
the amino acid sequence set forth in SEQ ID NO: 26, or an amino acid sequence having at least 90%, 91%, 92%, 93%, 94%, 95%, 96%, 97%, 98%, or 99% sequence identity to the amino acid sequence set forth in SEQ ID NO: 26 and having a substitution to cysteine at position 155, or an amino acid sequence having at least 90%, 91%, 92%, 93%, 94%, 95%, 96%, 97%, 98%, or 99% sequence identity to the amino acid sequence set forth in SEQ ID NO: 26 and comprising the amino acid sequence set forth in SEQ ID NO: 11; or
the modified heavy chain constant region comprises a modified CH1, and the modified CH1 comprises or consists of the following amino acid sequences:
the amino acid sequence set forth in SEQ ID NO: 22, or an amino acid sequence having at least 90%, 91%, 92%, 93%, 94%, 95%, 96%, 97%, 98%, or 99% sequence identity to the amino acid sequence set forth in SEQ ID NO: 22 and having a substitution to cysteine at position 152, or an amino acid sequence having at least 90%, 91%, 92%, 93%, 94%, 95%, 96%, 97%, 98%, or 99% sequence identity to the amino acid sequence set forth in SEQ ID NO: 22 and comprising the amino acid sequence set forth in SEQ ID NO: 10; or
the amino acid sequence set forth in SEQ ID NO: 23, or an amino acid sequence having at least 90%, 91%, 92%, 93%, 94%, 95%, 96%, 97%, 98%, or 99% sequence identity to the amino acid sequence set forth in SEQ ID NO: 23 and having a substitution to cysteine at position 155, or an amino acid sequence having at least 90%, 91%, 92%, 93%, 94%, 95%, 96%, 97%, 98%, or 99% sequence identity to the amino acid sequence set forth in SEQ ID NO: 23 and comprising the amino acid sequence set forth in SEQ ID NO: 11;
in item (iv) the modified light chain constant region comprises or consists of the following amino acid sequences: the amino acid sequence set forth in SEQ ID NO: 17, or an amino acid sequence having at least 90%, 91%, 92%, 93%, 94%, 95%, 96%, 97%, 98%, or 99% sequence identity to the amino acid sequence set forth in SEQ ID NO: 17 and having a substitution to cysteine at position 166, or an amino acid sequence having at least 90%, 91%, 92%, 93%, 94%, 95%, 96%, 97%, 98%, or 99% sequence identity to the amino acid sequence set forth in SEQ ID NO: 17 and comprising the amino acid sequence set forth in SEQ ID NO: 8; and
the modified heavy chain constant region comprises or consists of the following amino acid sequences:
the amino acid sequence set forth in SEQ ID NO: 25, or an amino acid sequence having at least 90%, 91%, 92%, 93%, 94%, 95%, 96%, 97%, 98%, or 99% sequence identity to the amino acid sequence set forth in SEQ ID NO: 25 and having a substitution to cysteine at position 152, or an amino acid sequence having at least 90%, 91%, 92%, 93%, 94%, 95%, 96%, 97%, 98%, or 99% sequence identity to the amino acid sequence set forth in SEQ ID NO: 25 and comprising the amino acid sequence set forth in SEQ ID NO: 10; or
the amino acid sequence set forth in SEQ ID NO: 26, or an amino acid sequence having at least 90%, 91%, 92%, 93%, 94%, 95%, 96%, 97%, 98%, or 99% sequence identity to the amino acid sequence set forth in SEQ ID NO: 26 and having a substitution to cysteine at position 155, or an amino acid sequence having at least 90%, 91%, 92%, 93%, 94%, 95%, 96%, 97%, 98%, or 99% sequence identity to the amino acid sequence set forth in SEQ ID NO: 26 and comprising the amino acid sequence set forth in SEQ ID NO: 11; or
the modified heavy chain constant region comprises a modified CH1, and the modified CH1 comprises or consists of the following amino acid sequences:
the amino acid sequence set forth in SEQ ID NO: 22, or an amino acid sequence having at least 90%, 91%, 92%, 93%, 94%, 95%, 96%, 97%, 98%, or 99% sequence identity to the amino acid sequence set forth in SEQ ID NO: 22 and having a substitution to cysteine at position 152, or an amino acid sequence having at least 90%, 91%, 92%, 93%, 94%, 95%, 96%, 97%, 98%, or 99% sequence identity to the amino acid sequence set forth in SEQ ID NO: 22 and comprising the amino acid sequence set forth in SEQ ID NO: 10; or
the amino acid sequence set forth in SEQ ID NO: 23, or an amino acid sequence having at least 90%, 91%, 92%, 93%, 94%, 95%, 96%, 97%, 98%, or 99% sequence identity to the amino acid sequence set forth in SEQ ID NO: 23 and having a substitution to cysteine at position 155, or an amino acid sequence having at least 90%, 91%, 92%, 93%, 94%, 95%, 96%, 97%, 98%, or 99% sequence identity to the amino acid sequence set forth in SEQ ID NO: 23 and comprising the amino acid sequence set forth in SEQ ID NO: 11; or
in item (v) the modified light chain constant region comprises or consists of the following amino acid sequences: the amino acid sequence set forth in SEQ ID NO: 20, or an amino acid sequence having at least 90%, 91%, 92%, 93%, 94%, 95%, 96%, 97%, 98%, or 99% sequence identity to the amino acid sequence set forth in SEQ ID NO: 20 and having a substitution to cysteine at position 165, or an amino acid sequence having at least 90%, 91%, 92%, 93%, 94%, 95%, 96%, 97%, 98%, or 99% sequence identity to the amino acid sequence set forth in SEQ ID NO: 20 and comprising the amino acid sequence set forth in SEQ ID NO: 9; and
the modified heavy chain constant region comprises or consists of the following amino acid sequences:
the amino acid sequence set forth in SEQ ID NO: 25, or an amino acid sequence having at least 90%, 91%, 92%, 93%, 94%, 95%, 96%, 97%, 98%, or 99% sequence identity to the amino acid sequence set forth in SEQ ID NO: 25 and having a substitution to cysteine at position 152, or an amino acid sequence having at least 90%, 91%, 92%, 93%, 94%, 95%, 96%, 97%, 98%, or 99% sequence identity to the amino acid sequence set forth in SEQ ID NO: 25 and comprising the amino acid sequence set forth in SEQ ID NO: 10; or
the amino acid sequence set forth in SEQ ID NO: 26, or an amino acid sequence having at least 90%, 91%, 92%, 93%, 94%, 95%, 96%, 97%, 98%, or 99% sequence identity to the amino acid sequence set forth in SEQ ID NO: 26 and having a substitution to cysteine at position 155, or an amino acid sequence having at least 90%, 91%, 92%, 93%, 94%, 95%, 96%, 97%, 98%, or 99% sequence identity to the amino acid sequence set forth in SEQ ID NO: 26 and comprising the amino acid sequence set forth in SEQ ID NO: 11; or
the modified heavy chain constant region comprises a modified CH1, and the modified CH1 comprises or consists of the following amino acid sequences:
the amino acid sequence set forth in SEQ ID NO: 22, or an amino acid sequence having at least 90%, 91%, 92%, 93%, 94%, 95%, 96%, 97%, 98%, or 99% sequence identity to the amino acid sequence set forth in SEQ ID NO: 22 and having a substitution to cysteine at position 152, or an amino acid sequence having at least 90%, 91%, 92%, 93%, 94%, 95%, 96%, 97%, 98%, or 99% sequence identity to the amino acid sequence set forth in SEQ ID NO: 22 and comprising the amino acid sequence set forth in SEQ ID NO: 10; or
the amino acid sequence set forth in SEQ ID NO: 23, or an amino acid sequence having at least 90%, 91%, 92%, 93%, 94%, 95%, 96%, 97%, 98%, or 99% sequence identity to the amino acid sequence set forth in SEQ ID NO: 23 and having a substitution to cysteine at position 155, or an amino acid sequence having at least 90%, 91%, 92%, 93%, 94%, 95%, 96%, 97%, 98%, or 99% sequence identity to the amino acid sequence set forth in SEQ ID NO: 23 and comprising the amino acid sequence set forth in SEQ ID NO: 11;
in item (vi) the modified light chain constant region comprises or consists of the following amino acid sequences:
the amino acid sequence set forth in SEQ ID NO: 60, or an amino acid sequence having at least 90%, 91%, 92%, 93%, 94%, 95%, 96%, 97%, 98%, or 99% sequence identity to the amino acid sequence set forth in SEQ ID NO: 60 and having substitutions to cysteine at positions 160 and 166 and having a substitution to tyrosine at position 178, or an amino acid sequence having at least 90%, 91%, 92%, 93%, 94%, 95%, 96%, 97%, 98%, or 99% sequence identity to the amino acid sequence set forth in SEQ ID NO: 60 and comprising the amino acid sequence set forth in SEQ ID NO: 62; or
in item (vii) the modified light chain constant region comprises or consists of the following amino acid sequences: the amino acid sequence set forth in SEQ ID NO: 44, or an amino acid sequence having at least 90%, 91%, 92%, 93%, 94%, 95%, 96%, 97%, 98%, or 99% sequence identity to the amino acid sequence set forth in SEQ ID NO: 44 and having a substitution to cysteine at position 166, or an amino acid sequence having at least 90%, 91%, 92%, 93%, 94%, 95%, 96%, 97%, 98%, or 99% sequence identity to the amino acid sequence set forth in SEQ ID NO: 44 and comprising the amino acid sequence set forth in SEQ ID NO: 66;
and the modified heavy chain constant region comprises or consists of the following amino acid sequences:
the amino acid sequence set forth in SEQ ID NO: 61, or an amino acid sequence having at least 90%, 91%, 92%, 93%, 94%, 95%, 96%, 97%, 98%, or 99% sequence identity to the amino acid sequence set forth in SEQ ID NO: 61 and having a substitution to cysteine at position 375, or an amino acid sequence having at least 90%, 91%, 92%, 93%, 94%, 95%, 96%, 97%, 98%, or 99% sequence identity to the amino acid sequence set forth in SEQ ID NO: 61 and comprising the amino acid sequence set forth in SEQ ID NO: 67; or
the modified heavy chain constant region comprises a modified Fc region, and the modified Fc region comprises or consists of the following amino acid sequences:
the amino acid sequence set forth in SEQ ID NO: 63, or an amino acid sequence having at least 90%, 91%, 92%, 93%, 94%, 95%, 96%, 97%, 98%, or 99% sequence identity to the amino acid sequence set forth in SEQ ID NO: 63 and having a substitution to cysteine at position 375, or an amino acid sequence having at least 90%, 91%, 92%, 93%, 94%, 95%, 96%, 97%, 98%, or 99% sequence identity to the amino acid sequence set forth in SEQ ID NO: 63 and comprising the amino acid sequence set forth in SEQ ID NO: 67;
in item (viii) the modified heavy chain constant region comprises or consists of the following amino acid sequences:
the amino acid sequence set forth in SEQ ID NO: 64, or an amino acid sequence having at least 90%, 91%, 92%, 93%, 94%, 95%, 96%, 97%, 98%, or 99% sequence identity to the amino acid sequence set forth in SEQ ID NO: 64 and having substitutions to cysteine at position 239 and 375, or an amino acid sequence having at least 90%, 91%, 92%, 93%, 94%, 95%, 96%, 97%, 98%, or 99% sequence identity to the amino acid sequence set forth in SEQ ID NO: 64 and comprising the amino acid sequence set forth in SEQ ID NO:67 and SEQ ID NO: 68; or
the modified heavy chain constant region comprises a modified Fc region, and the modified Fc region comprises or consists of the following amino acid sequences:
the amino acid sequence set forth in SEQ ID NO: 65, or an amino acid sequence having at least 90%, 91%, 92%, 93%, 94%, 95%, 96%, 97%, 98%, or 99% sequence identity to the amino acid sequence set forth in SEQ ID NO: 65 and having a substitution to cysteine at position 375, or an amino acid sequence having at least 90%, 91%, 92%, 93%, 94%, 95%, 96%, 97%, 98%, or 99% sequence identity to the amino acid sequence set forth in SEQ ID NO: 65 and comprising the amino acid sequence set forth in SEQ ID NO:67 and SEQ ID NO: 68.

6. The antibody or antigen-binding fragment thereof according to any one of claims 1-5, wherein the antigen-binding fragment is selected from Fab, Fab', F(ab')2 or diabody.

7. The antibody or antigen-binding fragment thereof according to any one of claims 1-6, wherein the antibody or antigen-binding fragment thereof specifically binds to an antigen, for example, a tumor-associated antigen, such as an immune checkpoint molecule, such as HER2.

8. The antibody or antigen-binding fragment thereof according to claim 7, wherein the antibody or antigen-binding fragment thereof that specifically binds to HER2 comprises three heavy chain variable region CDRs and three light chain variable region CDRs of a known antibody that specifically binds to HER2, such as trastuzumab; or comprises a heavy chain variable region and a light chain variable region of a known antibody that specifically binds to HER2, such as trastuzumab.

9. The antibody or antigen-binding fragment thereof according to claim 7, the parent antibody or antigen-binding fragment thereof is trastuzumab or an antigen-binding fragment thereof.

10. An isolated nucleic acid molecule encoding the antibody or antigen-binding fragment thereof according to any one of claims 1-9.

11. An expression vector comprising the nucleic acid molecule according to claim 10.

12. A host cell comprising the isolated nucleic acid molecule according to claim 10 or the expression vector according to claim 11.

13. A method for preparing the cysteine-engineered antibody or antigen-binding fragment thereof, comprising:
introducing a nucleic acid encoding each chain of the antibody or antigen-binding fragment thereof according to any one of claims 1-9 or an expression vector comprising said nucleic acid(s) into a host cell; and
expressing and assembling the antibody or antigen-binding fragment thereof in the host cell in vivo;
optionally, the antibody or antigen-binding fragment thereof is purified, e.g., by Protein A.

14. An immunoconjugate comprising the antibody or antigen-binding fragment thereof according to any one of claims 1-9 and a payload.

15. The immunoconjugate according to claim 14, wherein the payload is selected from a drug such as a small molecule drug, a radionuclide, DNA, RNA, an enzyme, or a polypeptide; such as a cytotoxic agent, a chemotherapeutic agent, an innate immune agonist (e.g., Toll-like receptor agonist (TLR) ISAC drugs SBT6050, SBT6290, BDC-1001; STING agonist ISAC drug XMT-2056, Treg cell regulatory ISAC drug ADCT-301, etc.), an immunomodulator, a therapeutic oligonucleotide (siRNA, PMO, etc.), or a radionuclide, etc.

16. The immunoconjugate according to claim 14, wherein the immunoconjugate is selected from an Antibody-Drug Conjugate (ADC), an Immunostimulatory Antibody Conjugate (ISAC), an Antibody-Oligonucleotide Conjugate (AOC), an Antibody-Polypeptide Conjugate (APC), an antigody Radionuclide Drug Conjugate (RDC), or an antibody Degraducer Conjugate (ADeC).

17. The immunoconjugate according to claim 16, wherein the immunoconjugate is an Antibody-Drug Conjugate (ADC), and wherein the payload is a drug moiety selected from the group consisting of: a topoisomerase I inhibitor, such as a camptothecin derivative or a camptothecin analog (e.g., DXd); an anti-tubulin agent, such as auristatins (MMAF, MMAE), taxane analogs (e.g., epothilones A and B), maytansinoids (DM1, DM4), tubulysin and analogs thereof; a DNA-acting drug such as PBD, and the like.

18. The immunoconjugate according to claim 16 or 17, wherein the immunoconjugate is an Antibody-Drug Conjugate, and wherein the ADC has an average DAR value of 1-4.

19. The immunoconjugate according to any one of claims 14-18, wherein the antibody or antigen-binding fragment thereof is linked to a payload by the cysteine(s) introduced by the mutation(s), e.g., with or without a linker.

20. The immunoconjugate according to claim 19, wherein the linker comprises a functional group capable of reacting with a thiol group on a cysteine residue present on the antibody or antigen-binding fragment thereof to form a covalent bond, and the functional group is, for example, selected from the following groups: maleimide, iodoacetamide, bromoacetamide, vinylpyridine, disulfide, pyridyl disulfide, haloacetamide, α -haloacetyl, active esters such as succinimidyl esters, 4-nitrophenyl esters, pentafluorophenyl esters, tetrafluorophenyl esters, anhydrides, acyl chlorides, sulfonyl chlorides, isocyanates and isothiocyanates, preferably maleimide.

21. The immunoconjugate according to claim 19, wherein the linker is selected from a maleimido-caproyl-valine-alaine (mc-va) linker, a maleimidobutanoic acid-valine-citrulline (mb-vc) linker, or a maleimido-caproyl-glycine-glycine-phenylalanine-glycine linker (mc-GGFG).

22. The immunoconjugate of any one of claims 17-21, wherein the immunoconjugate comprises the antibody or antigen-binding fragment thereof as defined in any one of claims 7-9.

23. The immunoconjugate according to claim 22, wherein the drug moiety of the immunoconjugate is a topoisomerase I inhibitor, e.g., a camptothecin analog (e.g., a DX-8951 derivative DXd).

24. The immunoconjugate according to claim 22 or 23, wherein the linker of the immunoconjugate is a maleimido-caproyl-glycine-glycine-phenylalanine-glycine linker (mc-GGFG).

25. A pharmaceutical composition comprising the antibody or antigen-binding fragment thereof according to any one of claims 1-9 or the immunoconjugate according to any one of claims 14-24.

26. A pharmaceutical combination comprising the antibody or antigen-binding fragment thereof according to any one of claims 1-9 or the immunoconjugate according to any one of claims 14-24, and one or more other therapeutic agents.

27. An antibody or antigen-binding fragment thereof according to any one of claims 1-9 or an immunoconjugate according to any one of claims 14-24 for use in therapy.

28. An antibody or antigen-binding fragment thereof according to any one of claims 1-9 or an immunoconjugate according to any one of claims 14-24 for use in treating a tumor such as cancer.

29. A method of treating a disease, e.g., a tumor such as a cancer, wherein the method comprises administering the antibody or antigen-binding fragment thereof according to any one of claims 1-9 or the immunoconjugate according to any one of claims 14-24 or the pharmaceutical composition according to claim 25 to a subject in need thereof.

30. The method according to claim 29, wherein the method further comprises administering the antibody or antigen-binding fragment thereof according to any one of claims 1-9 or the immunoconjugate according to any one of claims 14-24 or the pharmaceutical composition according to claim 25 in combination with one or more other therapeutic agents, for example, the other therapeutic agents are various therapeutic agents for treating a tumor.
